# EUROPEAN PATENT SPECIFICATION

(11) **EP 3 316 873 B1**
(45) Date of publication and mention of the grant of the patent: **28.04.2021**
(21) Application number: 16736713.5
(22) Date of filing: 28.06.2016
(51) Int. Cl.: A61K 9/70, A61K 35/00, A61K 9/00

(54) **DEVICE FOR ORAL DELIVERY OF ACTIVE AGENTS**
VORRICHTUNG ZUR ORALEN VERABREICHUNG VON WIRKSTOFFEN
DISPOSITIF D'ADMINISTRATION ORALE DE PRINCIPES ACTIFS

(30) Priority: 30.06.2015 US 201562187186 P
(43) Date of publication of application: 09.05.2018
(73) Proprietor: Entrega Inc., Boston, MA 02116 (US)
(72) Inventor: BONNER, Daniel, Waltham, Massachusetts 02453 (US); GARDNER, Colin R., Concord, Massachusetts 01742 (US); JOZEFIAK, Thomas H., Belmont, Massachusetts 02478 (US); LOOSE, Christopher R., Cambridge, Massachusetts 02139 (US); LUCCHINO, David, Charlestown, Massachusetts 02129 (US); MILLER, Andrew Craig, East Walpole, Massachusetts 02032 (US); TRAVERSO, Carlo Giovanni, Newton, Massachusetts 02459 (US); VERMA, Ayush, Newton, Massachusetts 02459 (US); COURY, Arthur J., Boston, Massachusetts 02116 (US); TRAN, Peter, Boston, Massachusetts 02115 (US); JANTZ, John, Arlington, Massachusetts 02476 (US)
(74) Representative: Forrest, Stuart
(86) International application number: PCT/US2016/039745
(87) International publication number: WO 2017/004000

(56) References cited:
- WO-A2-00/41740
- WO-A2-2014/102741

## Description

Oral dosing of active agents is attractive for many reasons, including ease of administration and high patient compliance. However, for some active agents, such as poorly absorbed, sensitive (i.e., pH sensitive, enzyme-sensitive, and the like), and/or high molecular weight active agents, oral dosing may be less effective or ineffective for achieving sufficient blood concentration of the active agent as compared to alternative dosing strategies. For example, active agents such as proteins and other macromolecules may be enzymatically degraded in the gastrointestinal tract and/or may have limited transport across the intestinal epithelium.

One potential strategy for circumventing the hostile environment of the gastrointestinal tract is to alter the environment through the use of protease inhibitors and/or derivatization of agents with polyethylene glycol to prevent enzymatic degradation. Another potential strategy is to increase the permeability of the tissue in the gastrointestinal tract such that absorption of an agent increases. An agent may be formulated with an excipient that can, for example, open the tight junctions of the intestine to allow an agent to pass through the intestinal epithelium. A further approach to improving delivery of an agent in the gastrointestinal tract is to apply an enteric coating to the agent such that the agent is released in the lower gastrointestinal tract where absorption of proteins occurs more readily.

Several modifications of simple dosage systems including liposomes, micelles, microparticles, nanoparticles, nanocrystals, and the like, and combinations thereof have been used as agent carriers to overcome poor agent bioavailability and avoids the use of needles. For example, agent-loaded mucoadhesive micro- and nanoparticles that adhere to intestinal mucus (*i.e.*, intestinal cells are covered with a layer of mucus) have been used to prolong the migration time of the particles in the intestine and extend release of the drug. While significant progress has been made in the development of such mucoadhesive devices, a need remains for improved mucoadhesive devices that can be orally administered and effectively protect an agent (*e.g.*, a drug) dosage form to deliver (*e.g.*, uni-directionally) an agent to the intestines.

WO2014102741A2 discloses a pharmaceutical dosage form for delivery of, at a target site in a human or animal body, an active pharmaceutical ingredient (API), said pharmaceutical dosage form comprising a shell defining therein an outlet; a polymeric matrix having at least one API, the polymeric matrix received within the shell; and a swellable polymer body located within the shell, wherein the swellable polymer body is pH responsive, such that in use, exposure of the body to a medium of increasing pH causes an increase in swelling and an increase in volume of the swellable polymer body, which in turn, facilitates displacement of the matrix through the outlet toward the target site for delivery of the API.

WO0041740A2 discloses microfabricated, asymmetrical, reservoir-containing particles for use in the oral delivery of biopolymer therapeutic agents such as peptides, proteins and oligonucleotides.

In one aspect, the present invention relates to a device for the delivery of an agent to an intestinal site, the device comprising a backing element, a mucoadhesive layer for adhering the device to the intestinal site, and a reservoir comprising the agent, wherein the mucoadhesive layer comprises a polymer, an opposing surface having the capacity to adhere to the intestinal site, and a population of passageway(s) formed through the mucoadhesive layer and extending from the reservoir to the opposing surface for delivery of the agent from the reservoir to the intestinal site, the mucoadhesive layer having a thickness of about 50 nm to about 10 mm, wherein:
i) each of the passageway(s) has a minimum diameter greater than 10 microns, the diameter being determined by cryogenic scanning electron microscopy after 30 minutes of hydration at 20 °C in phosphate buffered saline at pH 6.5; or
ii) the device comprises a push element for induction of convective flow of the agent to the intestinal site, wherein the agent comprises molecules having a molecular weight of at least 100 Da, the push element comprises an osmagent, and said passageways are permeable to said molecules; or
iii) the agent comprises a population of particles having a weight average particle size, P_{avg}, and wherein the population has a number average minimum diameter, D_{avg}, of at least 10 microns, and the ratio of D_{avg} to P_{avg} is at least 2; or
iv) the agent comprises a population of particles having a weight average particle size, P_{avg}, of at least 50 nm, and wherein the population has at least one member having a minimum diameter of at least 10 microns.

One aspect of the present disclosure is a device for the delivery of an agent to an intestinal site, the device comprising a backing element, a mucoadhesive element for adhering the device to the intestinal site, and a reservoir comprising the agent. The mucoadhesive element comprises a polymer, an opposing surface having the capacity to adhere to the intestinal site, and a population of passageway(s) extending from the reservoir to the opposing surface for delivery of the agent from the reservoir to the intestinal site. Each of the passageway(s) has a minimum diameter greater than 0.1 microns, the diameter being determined by cryogenic scanning electron microscopy after 20 minutes of hydration at 20 °C in phosphate buffered saline at pH 6.5.

A further aspect of the present disclosure is a device for the delivery of an agent to an intestinal site, the device comprising a backing element, a mucoadhesive element for adhering the device to the intestinal site, a push element for induction of convective flow of the agent to the intestinal site, and a reservoir comprising the agent. The agent comprises molecules having a molecular weight of at least 100 Da. The push element comprises an osmagent. The mucoadhesive element comprises a polymer, an opposing surface having the capacity to adhere to the intestinal site, and a population of passageway(s) extending from the reservoir to the opposing surface for delivery of the agent from the reservoir to the intestinal site that are permeable to the molecules.

A further aspect of the present disclosure is a device for the delivery of an agent to an intestinal site, the device comprising a backing element, a mucoadhesive element for adhering the device to the intestinal site, and a reservoir comprising the agent. The agent comprises a population of particles having a weight average particle size, P_{avg}. The mucoadhesive element comprises a polymer, an opposing surface having the capacity to adhere to the intestinal site, and a population of passageway(s) extending from the reservoir to the opposing surface for the delivery of the agent from the reservoir to the intestinal site wherein the population has a number average minimum diameter, D_{avg}, of at least 0.1 microns, and the ratio of D_{avg} to P_{avg} is at least 2.

A further aspect of the present disclosure is a device for the delivery of an agent to an intestinal site, the device comprising a backing element, a mucoadhesive element for adhering the device to the intestinal site, and a reservoir comprising the agent. The mucoadhesive element comprises a polymer, an opposing surface having the capacity to adhere to the intestinal site, and a population of passageway(s) extending from the reservoir to the opposing surface for the delivery of the agent from the reservoir to the intestinal site. At least one passageway has a minimum diameter greater than 0.1 microns, the diameter determined by cryogenic scanning electron microscopy after 20 minutes of hydration at 20 °C in phosphate buffered saline at pH 6.5.

Other disclosed aspects, features and embodiments of the present disclosure will be, in part, discussed and, in part, apparent in the following description. The scope of the invention is defined by the claims. Any embodiments which fall outside the scope of the claims are presented for comparative purposes only.

### BRIEF DESCRIPTION OF THE FIGURES

FIG. 1 shows one embodiment of a device with a backing element, a reservoir, and a mucoadhesive element.
FIG. 2 shows one embodiment of a cross section of a device that is adhered a mucosa.
FIG. 3 shows another embodiment of a cross section of a device that is adhered to a mucosa.
FIG. 4 shows one embodiment of a device with a backing element, a reservoir, a mucoadhesive element, and passageways.
FIG. 5 shows one embodiment of a cross section of a device with a backing element, a reservoir, a mucoadhesive element, and passageways.
FIGS. 6A-6H shows different embodiments of the Opposing Surface of the mucoadhesive element, each having a population of passageways.
FIG. 7 shows one embodiment of the passageways having an irregular shape and a maximum diameter of an agent having a size that pass through the passageways.
FIG. 8 shows another embodiment of a cross section of a device with a backing element, a reservoir, a mucoadhesive element, and passageways.
FIG. 9 shows one embodiment of a cross section of a device having a backing element covering the reservoir.
FIG. 10 shows one embodiment of a cross section of a device having a backing element covering the reservoir and the mucoadhesive element.
FIG. 11 shows one embodiment of a device having a backing element covering a bottom surface of the reservoir.
FIG. 12 shows another embodiment of a device having a backing element covering a bottom surface of the reservoir.
FIG. 13 shows one embodiment of a device having a backing element covering the opposing surface of the mucoadhesive element.
FIG. 14 shows another embodiment of a device having a backing element covering the opposing surface of the mucoadhesive element.
FIG. 15 is a schematic depicting a disadvantage of prior art mucoadhesives , which have pores that close after hydration due to swelling.
FIG. 16 is a schematic depicting the mucoadhesive element of the instant disclosure having passageways that remain open after hydration.
FIG. 17 shows one embodiment of a device further including a push element.
FIG. 18 shows another embodiment of a device further including a push element.
FIG. 19 shows one embodiment of a device further including a barrier element.
FIG. 20 is a schematic depicting hydration of the push element after hydration of the reservoir to deliver the agent to a tissue.
FIG. 21 shows one embodiment of the push element subdivided into push element compartments.
FIG. 22 shows one embodiment of a first push element compartment swelling before a second push element compartment.
FIG. 23 shows comparative results for a Surgical Bioavailability Assay.

Other aspects, embodiments and features of the inventive subject matter will become apparent from the following detailed description when considered in conjunction with the accompanying drawing. The accompanying figures are schematic and are not intended to be drawn to scale. For purposes of clarity, not every element or component is labeled in every figure, nor is every element or component of each embodiment of the inventive subject matter shown where illustration is not necessary to allow those of ordinary skill in the art to understand the inventive subject matter.

### DEFINITIONS

"Agent" as used herein refers to an a pharmaceutical agent, a drug, a small molecule drug, a drug conjugate, a prodrug, an antibody or an antibody fragment, a nucleic acid, a protein, a peptide, a polysaccharide, a small inorganic molecule, a small organic molecule (e.g., with a molecular weight of about 500 Da), a metabolically activated agent (e.g., a metabolite), a nutrient, a supplement, and the like, unless specified otherwise.

"Average Dry Thickness," as used herein in connection with a mucoadhesive element shall mean the arithmetic mean calculated by averaging the Dry Thickness of at least 3, and preferably at least 5, representative locations spaced approximately evenly across the Opposing Surface.

"Average Wet Thickness," as used herein in connection with a mucoadhesive element shall mean the arithmetic mean calculated by averaging the Wet Thickness of at least 10, and preferably at least 20, representative locations spaced approximately evenly across the Opposing Surface.

"Average Passageway Size," as used herein in connection with a mucoadhesive element shall mean the arithmetic mean calculated by averaging the passageway size of at least 10, and preferably at least 20, representative passageways having exits spaced approximately evenly across the Opposing Surface.

"Biodegradable" as used herein refers to materials that, when introduced into the body of an individual, patient, or subject, is broken down by cellular machinery or chemical processes (e.g., hydrolysis) into components ("degradation products") that the body can either reuse or dispose of without significant toxic effect. In some instances, the degradation products may also be biocompatible.

"Convective flow" as used herein refers to the flow of a fluid containing a diluent (e.g., a solvent) and a solute (e.g., an agent) wherein the diluent and solute flow at a substantially equivalent rate with respect to one another.

"Dry Thickness" as used in connection with a mucoadhesive element is the thickness of the mucoadhesive element measured from and in a direction perpendicular to the Opposing Surface of the mucoadhesive element at 20 °C via scanning electron microscopy (SEM).

"Individual," "patient," or "subject" as used herein are used interchangeably and refer to any animal, including mammals, preferably mice, rats, guinea pigs, and other rodents; rabbits; dogs; cats; swine; cattle; sheep; horses; birds; reptiles; or primates, most preferably, humans.

"Minimum Diameter" as used herein is the smallest internal diameter of an individual passageway at any point along the length of the passageway between the entrance and the exit thereof.

"Mucoadhesive" as used herein refers to a composition having the capacity to bind to a mucosal surface.

"Opposing Surface" as used in connection with the mucoadhesive element is the surface of the mucoadhesive element that opposes the tissue when a device of the present disclosure is adhered to a target tissue.

"Passageway Size," as used herein shall mean the diameter of the largest possible circle that may pass through the exit of the passageway within a representative region of the Opposing Surface.

"Pharmaceutically acceptable carrier" or "pharmaceutically acceptable excipient" as used herein refers to any and all solvents, dispersion media, coatings, isotonic and absorption delaying agents, and the like, that are Biocompatible and otherwise suitable for administration to an Individual.

"Pharmaceutical composition" as used herein refers to a composition comprising at least one agent as disclosed herein formulated together with one or more pharmaceutically acceptable carriers and/or excipients as disclosed herein.

"Pharmaceutically or pharmacologically acceptable" as used herein refers to molecular entities and compositions that do not produce an adverse, allergic, or other untoward reaction when administered to an animal, or a human, as appropriate.

"Swelling Ratio" as used herein in connection with a mucoadhesive element shall mean a ratio of the Average Wet Thickness to the Average Dry Thickness of a mucoadhesive element.

"Treating" as used herein refers to any effect, for example, lessening, reducing, modulating, or eliminating, that results in the improvement of the condition, disease, disorder, and the like.

"Wet Thickness" as used in connection with a mucoadhesive element is the thickness of the mucoadhesive element measured from and in a direction perpendicular to the Opposing Surface of the mucoadhesive element after 20 minutes of hydration at 20 °C in phosphate buffered saline at pH 6.5.

The singular forms "a," "an," and "the," as used herein, include plural referents unless the context clearly dictates otherwise.

The terms "comprising," "comprises," "including," and "includes" should be interpreted as referring to elements, components, or steps in a non-exclusive manner, indicating that the referenced elements, components, or steps may be present, utilized, or combined with other elements, components, or steps that are not expressly referenced.

### DETAILED DESCRIPTION

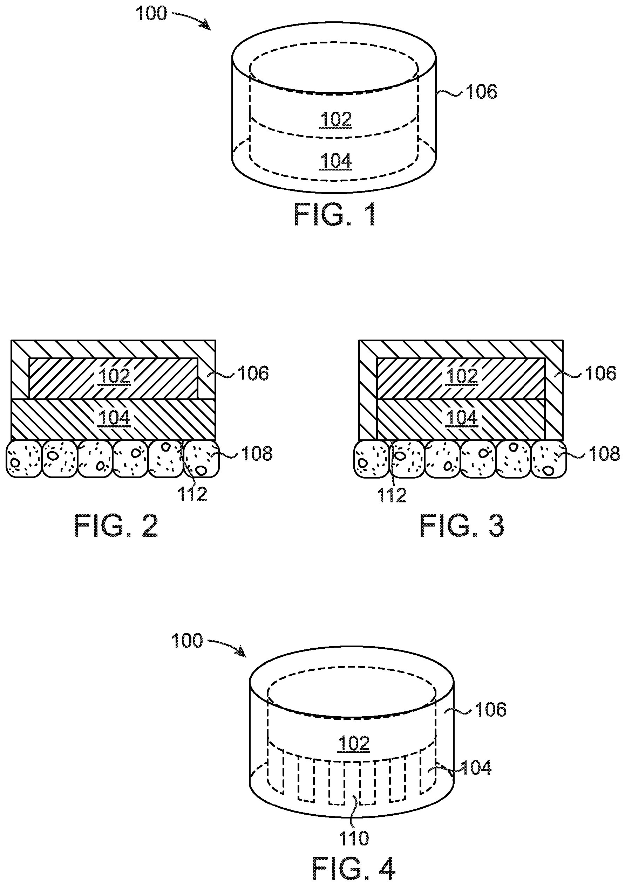

In general, the present disclosure is directed to devices, systems, and methods for the oral, trans-intestinal, and/or trans-mucosal delivery of an agent. As shown in FIG. 1, the device 100 comprises a reservoir 102 containing the agent, a mucoadhesive element 104 for adhering the device to a target tissue, and a backing element 106 that inhibits the unintended loss of the agent from the device. More specifically, the device is adapted to deliver the agent from the reservoir 102 to a target mucosa after the mucoadhesive element 104 is adhered to the target tissue while minimizing unintended and non-specific loss of agent to the surrounding environment (i.e., regions other than the surface of the target tissue to which the mucoadhesive device is adhered). Further, in general, a surface of the reservoir 102 may be directly adjacent to a surface of the mucoadhesive element 104, or a surface of the reservoir 102 may be directly adjacent to a surface of the backing element 106.

### Target Tissue

Typically, the target tissue is a tissue within the gastrointestinal tract such as, for example, the upper gastrointestinal tract or the lower gastrointestinal tract (i.e., the small intestine or large intestine). Referring now to FIGS. 2 and 3, the device 100 may adhere to a mucosa 108 at a location between the pyloric sphincter and the rectum. For instance, in one such embodiment, the device 100 may adhere to the mucosa 108 of the small intestine (e.g., the duodenum, jejunum, or ileum) and/or the large intestine (e.g., the ascending colon, the right colic flexure, the transverse colon, the transverse mesocolon, the left colic flexure, the descending colon, the sigmoid colon, and the rectum). In some embodiments, the device 100 may be capable of inserting into an invagination of an intestinal membrane.

The device 100 is adapted to adhere to a mucosa 108 and remain adhered for a period of time. For example, in some instances, the device 100 may adhere to a mucosa 108 for less than about 12 hours. By way of further example, in one embodiment, the device 100 may adhere to a mucosa 108 for less than about 4 hours. By way of further example, in one embodiment, the device 100 may adhere to a mucosa 108 for between about 30 minutes and about 12 hours. By way of further example, in one embodiment, the device 100 may adhere to a mucosa 108 for between about 30 minutes and about 4 hours. By way of further example, in one embodiment, the device 100 may adhere to a mucosa 108 for between about 1 hour and about 12 hours. By way of further example, in one embodiment, the device 100 may adhere to a mucosa 108 for between about 2 hours and about 12 hours. By way of further example, in one embodiment, the device 100 may adhere to a mucosa 108 for between about 2 hours and about 6 hours. By way of further example, in one embodiment, the device 100 may adhere to a mucosa 108 for between about 3 hours and about 4 hours. In certain embodiments, the device 100 may adhere to a mucosa 108 for at least about 30 minutes. By way of further example, in one embodiment, the device 100 may adhere to a mucosa 108 for at least about 1 hour. By way of further example, in one embodiment, the device 100 may adhere to a mucosa 108 for at least about 2 hours. By way of further example, in one embodiment, the device 100 may adhere to a mucosa 108 for at least about 3 hours. By way of further example, in one embodiment, the device 100 may adhere to a mucosa 108 for at least about 6 hours. By way of further example, in one embodiment, the device 100 may adhere to a mucosa 108 for at least about 12 hours.

### Agents

The device 100 of the present disclosure is adapted to deliver any of a wide range of agents to a tissue site. Thus, for example, the device 100 may be adapted to deliver a single agent or multiple agents (*e.g.*, two, three or more agents, either serially or simultaneously) to the tissue site after the device 100 is adhered thereto. Additionally, the agents may be in any of a wide range of alternative forms such as alternative salt forms, free acid forms, free base forms, and hydrates.

In general, the agent may be in particulate, liquid, or gel form and may comprise any of a range of compositions having biological relevance, e.g., metals, metal oxides, peptides, antibodies, hormones, enzymes, growth factors, small organic molecules ligands, nanoparticles that have surface activation, PEG, zwitterionic or targeting agents, or other pharmaceuticals, nutraceuticals, or biologics. In some embodiments, the agent(s) comprised by the reservoir 102 include one or more large molecules (*e.g.*, proteins and/or protein conjugates), and/or one or more small molecules (*e.g.*, small organic or inorganic molecules) as the agent(s). In one exemplary embodiment, the reservoir 102 comprises at least one agent selected from molecules having a molecular weight of at least 100 Da. By way of further example, in one embodiment, the reservoir 102 comprises at least one agent selected from molecules having a molecular weight of at least 200 Da. By way of further example, in one embodiment, the reservoir 102 comprises at least one agent selected from molecules having a molecular weight of at least 500 Da. By way of further example, in one embodiment, the reservoir 102 comprises at least one agent selected from molecules having a molecular weight of at least 1,000 Da. By way of further example, in one embodiment, the reservoir 102 comprises at least one agent selected from molecules having a molecular weight of at least 2,000 Da. By way of further example, in one embodiment, the reservoir 102 comprises at least one agent selected from molecules having a molecular weight of at least 5,000 Da. By way of further example, in one embodiment, the reservoir 102 comprises at least one agent selected from molecules having a molecular weight of at least 10,000 Da. By way of further example, in one embodiment, the reservoir 102 comprises at least one agent selected from molecules having a molecular weight of at least 50,000 Da. By way of further example, in one embodiment, the reservoir 102 comprises at least one agent selected from molecules having a molecular weight of at least 100,000 Da. By way of further example, in one embodiment, the reservoir 102 comprises at least one agent selected from molecules having a molecular weight of at least 500,000 Da. In general, however, the agent will typically have a molecular weight less than about 20,000 Da. As previously noted, the reservoir 102 may contain two or more agents independently selected from molecules having a molecular weight within the ranges recited within this paragraph.

The agent comprised by the reservoir 102 (or each agent in those embodiments in which the device 100 comprises more than one agent) in any suitable amount or concentration. For example, in one embodiment, the agent may be at least about 1% of the weight of the device 100. By way of further example, in one embodiment, the agent may be at least about 2% of the weight of the device 100. By way of further example, in one embodiment, the agent may be at least about 5% of the weight of the device 100. By way of further example, in one embodiment, the agent may be at least about 10% of the weight of the device 100. By way of further example, in one embodiment, the agent may be at least about 20% of the weight of the device 100. By way of further example, in one embodiment, the agent may be at least about 50% of the weight of the device 100. In certain embodiments, the agent may be between about 1% and about 50% of the weight of the device 100. By way of further example, in one embodiment, the agent may be between about 1% and about 20% of the weight of the device 100. By way of further example, in one embodiment, the agent may be between about 1% and about 10% of the weight of the device 100. By way of further example, in one embodiment, the agent may be between about 2% and about 50% of the weight of the device 100. By way of further example, in one embodiment, the agent may be between about 5% and about 50% of the weight of the device.

In one embodiment, the agent(s) is/are in the form of a population of particles having a weight average particle size, P_{avg}, of at least 1 nm. For example, in one such embodiment, the population has a weight average particle size, P_{avg}, of at least 5 nm. By way of further example, in one such embodiment the population has a weight average particle size, P_{avg}, of at least 10 nm. By way of further example, in one such embodiment the population has a weight average particle size, P_{avg}, of at least 50 nm. By way of further example, in one such embodiment the population has a weight average particle size, P_{avg}, of at least 100 nm. By way of further example, in one such embodiment the population has a weight average particle size, P_{avg}, of at least 200 nm. By way of further example, in one such embodiment the population has a weight average particle size, P_{avg}, of at least 500 nm. By way of further example, in one such embodiment the population has a weight average particle size, P_{avg}, of at least 1000 nm. In general, however, any such population will have an average size of less than 250 nm.

In each of the foregoing embodiments, the population of particles (comprising any of the previously mentioned compositions and having any of the previously described average sizes) may comprise particles that are a composite of two or more materials. For example, in one such embodiment the population may comprise composite particles wherein the composite particles comprise two or more components disposed in a core-shell arrangement; *e.g.*, one or more of the components is present within a core of the particles and surrounded by a shell of one or more other components. By way of further example, in another such embodiment one or more of the components is present as a dispersed discontinuous phase within a continuous phase of one or more components.

In each of the foregoing embodiments in which the agent is in the form of a population of particles, the population (e.g., composite particles or particles having a uniform composition, or mixture thereof) may be prepared using, for example, spray drying, interfacial polymerization, hot melt encapsulation, phase separation encapsulation, spontaneous emulsion, solvent evaporation microencapsulation, solvent removal microencapsulation, coacervation, and low temperature microsphere formation techniques.

### Mucoadhesive element

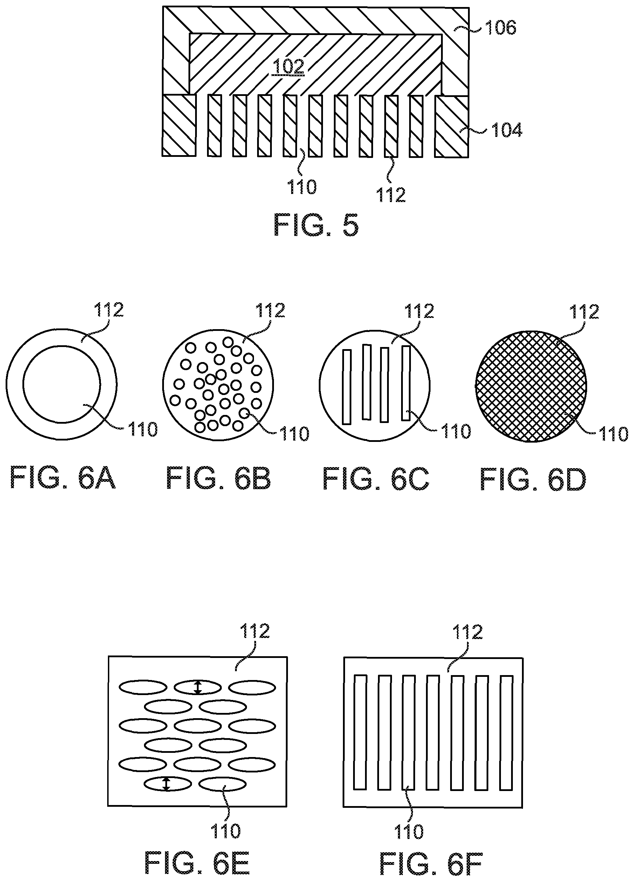

In general, the mucoadhesive element 104 is adapted to adhere to a target tissue and facilitate the delivery of the agent(s) from the device reservoir 102 to the target tissue. To facilitate this delivery, the mucoadhesive element 104 contains a population of passageways that provide a route for the flow of agent from the device reservoir to the surface of the tissue to which the device is adhered. As shown in FIGS. 4 and 5, in general, the passageway(s) 110 have a passageway entrance proximate the reservoir 102 and an exit proximate the surface of the mucoadhesive element 104 that opposes the tissue when the device 100 is adhered thereto.

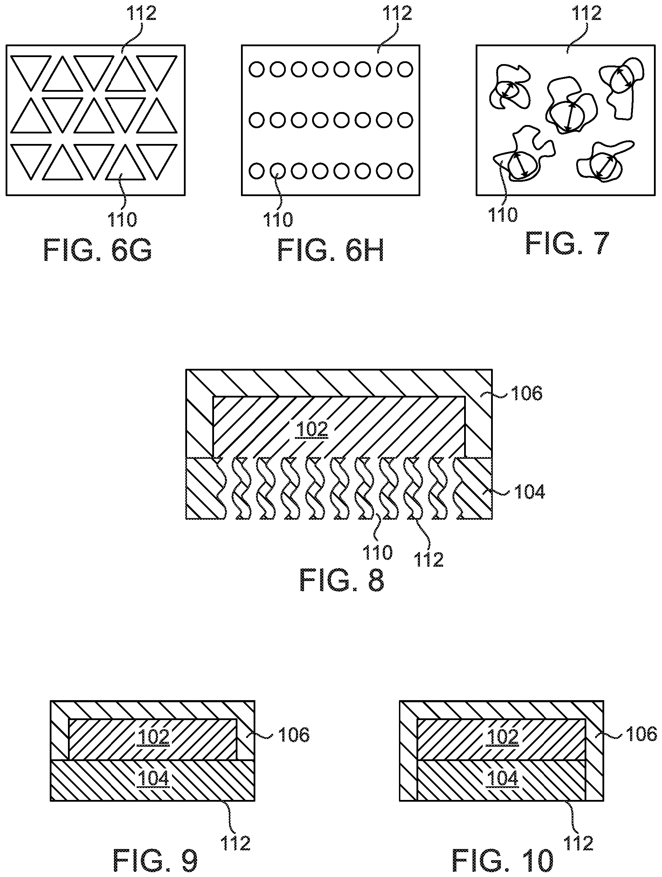

The surface 112 of the mucoadhesive element 104 that is adapted to oppose the tissue when the device is adhered to the issue (i.e., the opposing surface 112, as shown is FIGS. 6A-6H) may constitute a significant fraction of the total surface area of the device 100 (exposed to the surrounding environment). In general, the opposing surface 112 comprises at least 1% of the total surface area of the device 100 and may comprise as much as 99% of the total surface area of the device 100. For example, in one embodiment the opposing surface 112 comprises 10% to 95% of the total surface area of the device 100. By way of further example, in one such embodiment the opposing surface 112 comprises 15% to 90% of the total surface area of the device 100. By way of further example, in one such embodiment the opposing surface 112 comprises 20% to 90% of the total surface area of the device 100. By way of further example, in one such embodiment the opposing surface 112 comprises 20% to 85% of the total surface area of the device 100. By way of further example, in one such embodiment the opposing surface 112 comprises 25% to 85% of the total surface area of the device 100. By way of further example, in one such embodiment the opposing surface 112 comprises 25% to 80% of the total surface area of the device 100. By way of further example, in one such embodiment the opposing surface 112 comprises 30% to 85% of the total surface area of the device 100. By way of further example, in one such embodiment the opposing surface 112 comprises 30% to 80% of the total surface area of the device 100. By way of further example, in one such embodiment the opposing surface 112 comprises 30% to 75% of the total surface area of the device 100. By way of further example, in one such embodiment the opposing surface 112 comprises 40% to 60% of the total surface area of the device 100.

The mucoadhesive element 104 may have any of a wide range of geometric shapes (when viewed in a direction normal to the opposing surface 112). For example, the mucoadhesive element 104 may have an elliptical, circular or polygonal geometric shape when viewed in a direction that is perpendicular to the opposing surface 112.

The mucoadhesive element 104 may also have a range of Average Dry Thickness values. For example, in one embodiment, the mucoadhesive element 104 may have an Average Dry Thickness of less than about 1 micron. By way of further example, in one embodiment, the mucoadhesive element 104 may have an Average Dry Thickness of less than about 100 microns. By way of further example, in one embodiment, the mucoadhesive element 104 may have an Average Dry Thickness of less than about 200 microns. By way of further example, in one embodiment, the mucoadhesive element 104 may have an Average Dry Thickness of less than about 300 microns. By way of further example, in one embodiment, the mucoadhesive element 104 may have an Average Dry Thickness of less than about 400 microns. By way of further example, in one embodiment, the mucoadhesive element 104 may have an Average Dry Thickness of less than about 500 microns. By way of further example, in one embodiment, the mucoadhesive element 104 may have an Average Dry Thickness of less than about 600 microns. By way of further example, in one embodiment, the mucoadhesive element 104 may have an Average Dry Thickness of less than about 700 microns. By way of further example, in one embodiment, the mucoadhesive element 104 may have an Average Dry Thickness of less than about 800 microns. By way of further example, in one embodiment, the mucoadhesive element 104 may have an Average Dry Thickness of less than about 900 microns. By way of further example, in one embodiment, the mucoadhesive element 104 may have an Average Dry Thickness of less than about 1 mm. By way of further example, in one embodiment, the mucoadhesive element 104 may have an Average Dry Thickness of less than about 2 mm. By way of further example, in one embodiment, the mucoadhesive element 104 may have an Average Dry Thickness of less than about 5 mm. By way of further example, in one embodiment, the mucoadhesive element 104 may have an Average Dry Thickness of less than about 10 mm.

In certain embodiments of the present disclosure, swelling of the mucoadhesive element 104 in certain environments is controlled. For example, in one such embodiment, the mucoadhesive element 104 has a Swelling Ratio that does not exceed 5. By way of further example, in one such embodiment the mucoadhesive element 104 has a Swelling Ratio that does not exceed 4. By way of further example, in one such embodiment the mucoadhesive element 104 has a Swelling Ratio that does not exceed 3. By way of further example, in one such embodiment the mucoadhesive element 104 has a Swelling Ratio that does not exceed 2. By way of further example, in one such embodiment the mucoadhesive element 104 has a Swelling Ratio that does not exceed 1.5. By way of further example, in one such embodiment the mucoadhesive element 104 has a Swelling Ratio that does not exceed 1.3. By way of further example, in one such embodiment the mucoadhesive element 104 has a Swelling Ratio that does not exceed 1.2. By way of further example, in one such embodiment the mucoadhesive element 104 has a Swelling Ratio that does not exceed 1.1. By way of further example, in one such embodiment the mucoadhesive element104 has a Swelling Ratio that does not exceed 1.05. By way of further example, in one such embodiment the mucoadhesive element 104 has a Swelling Ratio that does not exceed 1.

The number of members in the passageway population that pass through the mucoadhesive element 104 may depend upon a range of factors including, for example, size of agent, viscosity of reservoir when hydrated, and desired release time. Referring now to FIG. 6A, in one embodiment, the population of passageways 110 has only one member; that is, the mucoadhesive element 104 comprises a single passageway 110. In other embodiments, the population comprises two or more members; that is, the mucoadhesive element 104 comprises two or more passageways 110, as shown in FIGS. 6B-6H. In still yet another embodiment, the population of passageways may include greater than 1000 members. By way of further example, in one embodiment, the population of passageways may include greater than 100 members. By way of further example, in one embodiment, the population of passageways may include great than 20 members. By way of further example, in one embodiment, the population of passageways may include great than 5 members. By way of further example, in one embodiment, the population of passageways may include greater than 1 member.

As previously noted, the passageway(s) 110 provide a path for delivery of the agent(s) comprised by the reservoir 102 to the tissue. As shown in FIG. 7, in some embodiments, therefore, the passageway(s) 110 have a bore (inner diameter) sufficient to permit the passage of particles having a particle size of at least 50 nm from the entrance and to the exit of the passageway(s) (i.e., from the reservoir 102 to the surface 112 of the mucoadhesive element 104 that opposes the tissue when the device 100 is adhered to the tissue); stated differently, in one embodiment, the passageway(s) 110 permit the passage of particles having a size of at least 50 nm as measured by dynamic light scattering. For example, in one such embodiment the passageway(s) 110 are permeable to particles having a size of at least 1 nm. By way of further example, in one such embodiment the passageway(s) 110 are permeable to particles having a size of at least 5 nm. By way of further example, in one such embodiment the passageway(s) 110 are permeable to particles having a size of at least 100 nm. By way of further example, in one such embodiment the passageway(s) 110 are permeable to particles having a size of at least 200 nm.

To facilitate delivery of agent(s) having a range of sizes to the tissue, it is generally preferred that at least one member of the population of passageway(s) 110 extending from the reservoir 102 to the opposing surface 112 have a minimum diameter of at least 10 microns. For example, in one such embodiment at least one (and preferably each) member of the population of passageway(s) 110 extending from the reservoir 102 to the opposing surface 112 have a minimum diameter of at least 15 microns. By way of further example, in one such embodiment at least one (and preferably each) member of the population of passageway(s) 110 extending from the reservoir 102 to the opposing surface 112 have a minimum diameter of at least 20 microns. By way of further example, in one such embodiment at least one (and preferably each) member of the population of passageway(s) 110 extending from the reservoir 102 to the opposing surface 112 have a minimum diameter of at least 25 microns. By way of further example, in one such embodiment at least one (and preferably each) member of the population of passageway(s) 110 extending from the reservoir 102 to the opposing surface 112 have a minimum diameter of at least 50 microns. By way of further example, in one such embodiment at least one (and preferably each) member of the population of passageway(s) 110 extending from the reservoir 102 to the opposing surface 112 have a minimum diameter of at least 75 microns. By way of further example, in one such embodiment at least one (and preferably each) member of the population of passageway(s) 110 extending from the reservoir 102 to the opposing surface 112 have a minimum diameter of at least 100 microns. By way of further example, in one such embodiment at least one (and preferably each) member of the population of passageway(s) 110 extending from the reservoir 102 to the opposing surface 112 have a minimum diameter of at least 250 microns. By way of further example, in one such embodiment at least one (and preferably each) member of the population of passageway(s) 110 extending from the reservoir 102 to the opposing surface 112 have a minimum diameter of at least 500 microns. In each of the foregoing embodiments, the diameter may be determined by cryogenic scanning electron microscopy after 30 minutes of hydration at 20 °C in phosphate buffered saline at pH 6.5.

In one embodiment, it is preferred that the population of passageway(s) 110 extending from the reservoir 102 to the opposing surface 112 have a number average minimum diameter of at least 10 microns. For example, in one such embodiment at least one (and preferably each) member of the population of passageway(s) 110 extending from the reservoir 102 to the opposing surface 112 have a number average minimum diameter of at least 15 microns. By way of further example, in one such embodiment at least one (and preferably each) member of the population of passageway(s) 110 extending from the reservoir 102 to the opposing surface 112 have a number average minimum diameter of at least 20 microns. By way of further example, in one such embodiment at least one (and preferably each) member of the population of passageway(s) 110 extending from the reservoir 102 to the opposing surface 112 have a number average minimum diameter of at least 25 microns. By way of further example, in one such embodiment at least one (and preferably each) member of the population of passageway(s) 110 extending from the reservoir 102 to the opposing surface 112 have a number average minimum diameter of at least 50 microns. By way of further example, in one such embodiment at least one (and preferably each) member of the population of passageway(s) 110 extending from the reservoir 102 to the opposing surface 112 have a number average minimum diameter of at least 75 microns. By way of further example, in one such embodiment at least one (and preferably each) member of the population of passageway(s) 110 extending from the reservoir 102 to the opposing surface 112 have a number average minimum diameter of at least 100 microns. By way of further example, in one such embodiment at least one (and preferably each) member of the population of passageway(s) 110 extending from the reservoir 102 to the opposing surface 112 have a number average minimum diameter of at least 250 microns. By way of further example, in one such embodiment at least one (and preferably each) member of the population of passageway(s) 110 extending from the reservoir 102 to the opposing surface 112 have a number average minimum diameter of at least 500 microns. In each of the foregoing embodiments, the diameter may be determined by cryogenic scanning electron microscopy after 30 minutes of hydration at 20 °C in phosphate buffered saline at pH 6.5.

In one embodiment, the agent(s) is/are in the form of a population of particles having a weight average particle size, P_{avg}, and the population of passageways have a number average minimum diameter (D_{avg}) as previously described. For example, in one such embodiment, a ratio of D_{avg}:P_{avg} is at least 2. By way of further example, in one such embodiment, the ratio of D_{avg}:P_{avg} is at least 5. By way of further example, in one such embodiment, the ratio of D_{avg}:P_{avg} is at least 10. By way of further example, in one such embodiment, the ratio of D_{avg}:P_{avg} is at least 25. By way of further example, in one such embodiment, the ratio of D_{avg}:P_{avg} is at least 50. By way of further example, in one such embodiment, the ratio of D_{avg}:P_{avg} is at least 100. By way of further example, in one such embodiment, the ratio of D_{avg}:P_{avg} is at least 10,000. By way of further example, in one such embodiment, the ratio of D_{avg}:P_{avg} is at least 100,000. By way of further example, in one such embodiment, the ratio of D_{avg}:P_{avg} is at least 1,000,000. By way of further example, in one such embodiment, the ratio of D_{avg}:P_{avg} is at least 5,000,000.

In one embodiment, it is generally preferred that the exit of the members of the passageway population 110 have an Average Passageway Size of at least 1 mm, as depicted in FIG. 7. In certain embodiments, the Average Passageway Size may range from microns to millimeters. For example, in one embodiment, the passageway exit(s) have an Average Passageway Size of at least 0.1 microns. By way of further example, in one such embodiment the passageway exit(s) have an Average Passageway Size of at least 0.5 microns. By way of further example, in one such embodiment the passageway exit(s) have an Average Passageway Size of at least 1 micron. By way of further example, in one such embodiment the passageway exit(s) have an Average Passageway Size of at least 5 microns. By way of further example, in one such embodiment the passageway exit(s) have an Average Passageway Size of at least 10 microns. By way of further example, in one such embodiment the passageway exit(s) have an Average Passageway Size of at least 50 microns. By way of further example, in one such embodiment the passageway exit(s) have an Average Passageway Size of at least 0.1 mm. By way of further example, in one such embodiment the passageway exit(s) have an Average Passageway Size of at least 0.5 mm. By way of further example, in one such embodiment the passageway exit(s) have an Average Passageway Size of at least 1 mm. By way of further example, in one such embodiment the passageway exit(s) have an Average Passageway Size of at least 2 mm. By way of further example, in one such embodiment the passageway exit(s) have an Average Passageway Size of at least 2.5 mm. By way of further example, in one such embodiment the passageway exit(s) have an Average Passageway Size of at least 3 mm. Typically, however, the passageway exit(s) will have an Average Passageway Size of less than 5 mm. Thus, for example, the passageway exits will have an Average Passageway Size of less than 2 mm. By way of further example, in one such embodiment the passageway exit(s) have an Average Passageway Size of less than 1 mm.

Depending upon the composition of the mucoadhesive element 104, it may swell upon hydration and thus reduce the inner diameter of the passageways 110 (relative to the non-hydrated state) thus inhibiting the flow of agent from the reservoir 102 to the opposing surface 112. To compensate for this, the passageways 110 may be sized (oversized) in the non-hydrated state such that any reduction in the passageway diameter upon hydration does not significantly inhibit the flow of agent. Alternatively, the composition of the mucoadhesive element 104 may be selected such that the element does not significantly swell (e.g., has a low Swelling Ratio as described elsewhere herein). In a further alternative, the mucoadhesive element 104 may comprise a composite of two materials: a framework material (e.g., a metal or non-swelling polymer) that does not significantly swell upon hydration with an overcoat of a mucoadhesive polymer (at the opposing surface 112) to facilitate adhesion to the tissue, the overcoat being sufficiently thin that the mucoadhesive polymer would not occlude the openings in the framework upon hydration.

Independent of the number of members in the population, and independent of their dimensions, the path that the members trace between the reservoir 102 and the surface 112 of the mucoadhesive element 104 (adapted to adhere to the target tissue) may range from linear to highly tortuous. In certain embodiments, the members may trace a straight or linear path through the mucoadhesive element. For example, in one embodiment, the population of passageways 110 may include a straight or linear structure traversing from the reservoir 102 through the mucoadhesive element 104 to the opposing surface 112 of the mucoadhesive element 104 (i.e., the surface adapted for adhering to the tissue site), as shown in FIG. 5. By way of further example, in one embodiment, the population of passageways 110 may trace a non-linear or tortuous path from the reservoir 102 through the mucoadhesive element 104 to the opposing surface 112 of the mucoadhesive element 104. By way of further example, in one embodiment, the population of passageways 110 may trace a non-linear or wavy structure from the reservoir 102 through the mucoadhesive element 104 to the opposing surface 112 of the mucoadhesive element 104 as shown in FIG. 8. By way of further example, in one embodiment, the population of passageways 110 may trace a non-linear, dendritic, branched, irregular, or random structure(s) from the reservoir 102 through the mucoadhesive element 104 to the opposing surface 112 of the mucoadhesive element 104.

In one embodiment, the number, linearity and spacing of the members of the passageway population though the mucoadhesive material comprised by the mucoadhesive element 104 constitutes a mesh. For example, in one such embodiment, the openings in the mesh serve as the passageways 110 through which the agent may flow through, as shown in FIG. 6D.

In some embodiments, the mucoadhesive element 104 has surface features that may be measured using profilometry or light microscopy. For example, in one embodiment, the surface features include cylinders, ridges, and bumps. The features may have a dimension of at least 0.05 microns. By way of further example, the features may have a dimension of at least 0.5 microns. By way of further example, the features may have a dimension of at least 5 microns. By way of further example, the features may have a dimension of at least 50 microns. By way of further example, the features may have a dimension of at least 500 microns. By way of further example, the features may have a dimension of at least 1 mm.

In general, the mucoadhesive element 104 comprises a natural or synthetic polymer through which the passageway(s) pass. In some embodiments, the polymer may be biodegradable; in other embodiments, the polymer may be non-biodegradable. The polymer may be, for example, a homopolymer or a copolymer comprising the residues of two or more monomers. In terms of sequence, copolymers may be random, block, or include a combination of random and block sequences. Contemplated polymers may be biocompatible and/or biodegradable. The polymers described herein (i.e., for any purpose) may have any suitable molecular weight. In some embodiments, a polymer may have a molecular weight of between about 10 kDa to about 2000 kDa, in some embodiments between about 20 kDa and about 1000 kDa, in some embodiments between about 40 kDa and about 1000 kDa, and in some embodiments, between about 40 kDa and about 500 kDa.

In one embodiment, the polymer is a homopolymer or a copolymer comprising one or more natural polymer materials such as polysacharrides and oligosacharrides, further including both homo- and hetero-polysacharrides and oligosacharrides, respectively. By way of further example, in one embodiment, polysaccharrides include modified celluloses, pectins, chitosans, alginates, hyaluronates, xanthum gum, dextrans, *mefp-1* (aka Mussel Glue Protein), and the like. By way of further example, in one embodiment, modified celluloses include carboxymethyl cellulose, sodium carboxymethylcellulose hydroxymethyl propyl cellulose, hydroxypropyl methylcellulose, hydroxyethyl cellulose, hydroxypropyl cellulose, and the like. By way of further example, in one embodiment, chitosans include zinc-pectinate chitosan, calcium-chitosan, chemically modified chitosan, and the like. By way of further example, in one embodiment, chemically modified chitosan includes thiolated chitosan, chitosan bearing catechol functional groups, and the like. By way of further example, in one embodiment, hyaluronate includes sodium hyaluronate. By way of further example, in one embodiment, dextran includes DEAE-dextran, glucose, and the like. By way of further example, in one embodiment, oligosaccharrides include fructooligosaccharides (FOS), and the like. In some embodiments, each of the foregoing polymers may include mixtures of two or more foregoing polymers, copolymers, blends, and the like. For example, in one embodiment, copolymers include random, block, or a combination of random and block copolymer sequences. In another embodiment, for example, synthetic polymers include poly(2-acrylamido-2-methyl-1-propanesulfonic acid (PolyAMPS), polymers and copolymers of acrylic acid, methacrylic acid, and their lower alkyl esters; Carbopol ®; carbomer; polycarbophil; polyanhydrides; polyvinyl alcohols; polyvinylpyrrolidone (PVP); and the like. By way of further example, in one embodiment, polymers and copolymers of acrylic acid, methacrylic acid, and their lower alkyl esters include polyacrylic acid, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), and the like. By way of further example, in one embodiment, Carbopol® includes Carbopol® 934 (BF Goodrich Co., Cleveland, OH), and the like. In some embodiments, each of the foregoing polymers may include mixtures of two or more foregoing polymers, copolymers, blends, and the like. For example, in one embodiment, copolymers include random, block, or a combination of random and block copolymer sequences.

Non-limiting examples of biocompatible polymers that may be useful in various embodiments of the present disclosure include polydioxanone (PDO), polyhydroxyalkanoate, polyhydroxybutyrate, poly(glycerol sebacate), polyglycolide, polylactide, PLGA, PLA, polycaprolactone, or copolymers or derivatives including these and/or other polymers.

In some embodiments, the polymer may be degradable. For example, in one embodiment, a polymer may be one that hydrolyzes spontaneously upon exposure to water (e.g., within a subject). By way of further example, in one embodiment, the polymer may degrade upon exposure to heat (e.g., at temperatures of about 37 °C). Degradation of a polymer may occur at varying rates, depending on the polymer or copolymer used. For example, the half-life of the polymer (i.e., the time at which 50% of the polymer can be degraded into monomers and/or other non-polymeric moieties) may be on the order of days, weeks, months, or years, depending on the polymer. The polymers may be biologically degraded, for example, by enzymatic activity or cellular machinery, in some cases, for example, through exposure to a lysozyme (e.g., having relatively low pH).

In some cases, the polymers may be broken down into monomers and/or other nonpolymeric moieties that cells can either reuse or dispose of without significant toxic effect on the cells (e.g., polylactide may be hydrolyzed to form lactic acid; polyglycolide may be hydrolyzed to form glycolic acid; etc.).

In some embodiments, polymers may be polyesters, including copolymers having lactic acid and glycolic acid units, such as poly(lactic)-co-poly(glycolic) acid, poly(lactic acid-co-glycolic acid), and poly(lactide-co-glycolide), collectively referred to herein as "PLGA"; and homopolymers including glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some embodiments, exemplary polyesters include, polyhydroxyacids or polyanhydrides.

In other embodiments, polymers may be one or more acrylic polymers. In certain embodiments, acrylic polymers include, for example, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), methacrylic acid alkylamide copolymer, poly(methyl methacrylate), poly(methacrylic acid polyacrylamide, amino alkyl methacrylate copolymer, glycidyl methacrylate copolymers, polycyanoacrylates, and combinations including one or more of the foregoing polymers. The acrylic polymer may include fully-polymerized copolymers of acrylic and methacrylic acid esters with a low content of quaternary ammonium groups.

PLGA contemplated for use as described herein can be characterized by a lactic acid:glycolic acid ratio of, for example, approximately 85:15, approximately 75:25, approximately 60:40, approximately 50:50, approximately 40:60, approximately 25:75, or approximately 15:85. In some embodiments, the ratio of lactic acid to glycolic acid monomers in the polymer of the particle (e.g., a PLGA block copolymer or PLGA-PEG block copolymer), may be selected to optimize for various parameters such as water uptake, therapeutic agent release and/or polymer degradation kinetics can be optimized. In other embodiments, the end group of a PLA polymer chain may be a carboxylic acid group, an amine group, or a capped end group with, for example, a long chain alkyl group or cholesterol. Devices 100 disclosed herein may or may not contain polyethylene glycol.

### Backing Element

In general, the backing element 106 inhibits the release of the agent into the surrounding environment. Thus, the backing element 106 may have limited permeability or may be impermeable to, for example, an agent within the reservoir 102 of the device 100. In one exemplary embodiment, the backing element 106 may have limited permeability to the agent such that release of the agent is directed towards the mucosa resulting in an enhanced local concentration of the agent (e.g., the agent may pass through the mucus layer and through intestinal cells or between intestinal cells to enter the circulation system of the subject). In some embodiments, the backing element 106 may be selectively permeable. For example, the backing element 106 may be permeable to a fluid and impermeable to certain solutes dissolved in the fluid (e.g., proteins). By way of further example, in one embodiment, the backing element 106 may be permeable to water (e.g., water that is inside or outside of the device), but impermeable to proteins dissolved in the water (i.e., proteins dissolved in the water that are outside of the device). In some embodiments, the backing element 106 may at least partially prevent leakage of the agent from the reservoir 102 to the environment outside of the device. In some instances, the backing element 106 may at least partially prevent infiltration of undesirable molecules into the reservoir. For example, infiltration of undesirable molecules into the reservoir 102 may, for example, dilute the effective concentration of the agent in the reservoir 102, or may cause undesirable molecules to be delivered to a tissue site. By way of further example, the delivery of undesirable molecules to the tissue site may cause a toxic response. In one embodiment, the backing element 102 may be impermeable to the agent inside the device 100.

In some embodiments, the backing element 106 may include one or more layers or components. For example, in some instances, the backing element 106 may include a first layer or component that has limited permeability to a first class of molecules, and a second layer or component that has limited permeability to a second class of molecules. The permeability of the backing element 106 may, in some cases, be related to one or more properties of the molecules for which the backing element 106 has limited permeability. For example, the molecular weight and/or charge of a molecule may influence the permeability of the molecule through the backing element 106. In some embodiments, the backing element 106 may be essentially impermeable to molecules having a molecular weight above about 50 Da, in some embodiments above about 100 Da, in some embodiments above about 200 Da, in some embodiments above about 300 Da, in some embodiments above about 500 Da, in some embodiments above about 1000 Da, in some embodiments above about 2000 Da, in some embodiments above about 5000 Da, and in some embodiments above about 10000 Da.

The backing element 106 may include any suitable, biocompatible material. For example, in some cases, the backing element 106 may be a degradable or non-degradable material. By way of further example, in some embodiments, the backing element 106 may include a polymer. For example, in one embodiment, the polymer may be a natural polymer or a synthetic polymer. By way of further example, in some embodiments, the backing element may include poly(hydroxy acids), polyanhydrides, polyorthoesters, polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polysiloxanes, polyurethanes, derivatized celluloses, and polymers of acrylic acid, methacrylic acid or copolymers or derivatives thereof including acids and esters, and the like. Exemplary poly(hydroxy acids) include poly(lactic acid), poly(glycolic acid), polylactic acid-co-glycolic acid), and the like, and combinations thereof. Exemplary polyalkylenes include polyethylene, polypropylene, polystyrene, and the like, and combinations thereof. Exemplary polyalkylene glycols include poly(ethylene glycol), and the like. Exemplary polyalkylene oxides include poly(ethylene oxide), and the like. Exemplary polyalkylene terepthalates include poly(ethylene terephthalate), and the like. Exemplary polyvinyl esters include poly(vinyl acetate), and the like. Exemplary polyvinyl halides include poly(vinyl chloride), and the like. Exemplary derivatized celluloses include alkyl cellulose, hydroxyalkyl celluloses, cellulose ethers, cellulose esters, nitro celluloses, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxyethyl cellulose, cellulose triacetate, cellulose sulfate sodium salt (jointly referred to herein as "synthetic celluloses"), and the like. Exemplary polymers of acrylic acid, methacrylic acid or copolymers or derivatives thereof including acids may include poly(butyric acid), poly(valeric acid), and copolymers and blends thereof. Exemplary polymers of acrylic acid, methacrylic acid or copolymers or derivatives thereof including esters may include poly(methyl methacrylate), poly(ethyl methacrylate), polybutylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(lactide-co-caprolactone) (jointly referred to herein as "polyacrylic esters"), and copolymers and blends thereof. Exemplary degradable materials include biodegradable polymers, which include polymers of hydroxy acids such as lactic acid and glycolic acid, and copolymers with PEG, polyanhydrides, poly(ortho)esters, polyurethanes, poly(butyric acid), poly(valeric acid), poly(lactide-co-caprolactone), and blends and copolymers thereof. Exemplary non-degradable materials include non-biodegradable polymers, which include ethylene vinyl acetate, poly(meth)acrylic acid, polyamides, copolymers and blends thereof.

In some embodiments, one or more plasticizers may be added to the backing element. In one embodiment, the plasticizer may facilitate compliance of the backing element with the swelling of the device. In another embodiment, the plasticizer may facilitate compliance of the backing element 106 with the flexibility of the device 100. For example, in one embodiment, the backing element 106 may expand to allow for the protection of a privileged region. By way of further example, in one embodiment, plasticizers may include esters, multi-ring aromatic compounds, gases, alkyl sulfonates, chlorinated paraffins, energetic plasticizers, epoxides, glycol ethers and their esters, hydrocarbon oils, pentaerythritol derivatives, phosphates, polymeric plasticizers, polybutenes, sulfonamides, calcium stearate, fluorine-containing plasticizers, isocyanate adducts, natural product derivatives, nitrites, siloxane-based plasticizers, tar-based products, thioesters, glycerol, and the like. Exemplary esters include abietates, adipates, azelates, benzoates, citrates, glutarates, isobutyrates, oleates, phthalates, ricinoleates, sebacates, tri- and pyromellitates, difuran diesters, hydroxybenzoic acid esters, and the like. Exemplary multi-ring aromatic compounds include biphenyl derivatives, and the like. Exemplary gases include carbon dioxide, and the like. In one embodiment, the plasticizer may be glycerol at a concentration of about 2% w/v.

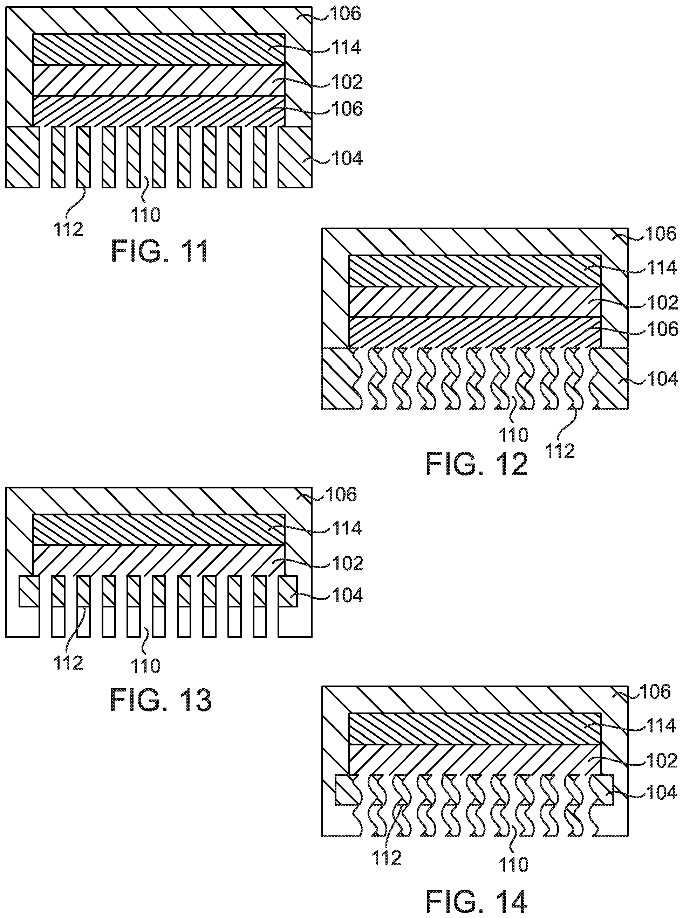

In some embodiments, the backing element 106 may envelope at least a portion of the reservoir 102, and/or optionally envelope at least a portion of the mucoadhesive element 104. For example, in one embodiment, the backing element 106 may envelope the upper surface and left and right lateral surfaces of the reservoir 102, as shown in FIG. 9. By way of further example, in one embodiment, the backing element 106 may envelope the upper surface and left and right lateral surfaces of the reservoir 102, and may also envelope the left and right lateral surfaces of the mucoadhesive element 104, as shown in FIG. 10. In other embodiments, the backing element 106 may envelope the bottom surface of the reservoir 102, as shown in FIGS. 11 and 12. In yet other embodiments, the backing element 106 may envelope the opposing surface of the mucoadhesive element 104, as shown in FIGS. 13 and 14.

In some embodiments, the backing element 106 may have reduced mucoadhesiveness relative to the opposing surface 112 of the mucoadhesive element 104 of the device 100, as described in more detail elsewhere herein. In other embodiments, the backing element 106 may be essentially non-mucoadhesive. In some embodiments, the backing element 106 may be degradable. In some instances, the backing element 106 may be more slowly degradable than other elements or components of the device. For example, in one embodiment, the backing element 106 may degrade more slowly compared to the mucoadhesive element 104. In certain embodiments, the backing element 106 may substantially protect the device's overall integrity after administration.

In some embodiments, the backing element 106 and the reservoir 102 may each swell at a rate relative to each other to sufficiently maintain the integrity of the device. In some embodiments, the backing element 106 and/or the reservoir 102 (and/or one or more other elements, for example, the mucoadhesive element 104) should be sufficiently flexible such that when the device 100 swells (e.g., due to water absorption), one or more elements or layers do not separate (e.g., delaminate). For example, in one embodiment, swelling or flexibility of the backing layer 106 and/or reservoir 102 should not cause separation of the backing layer 106 and/or reservoir 102 from one another, or separation of the reservoir 102 from the mucoadhesive element 104. As discussed elsewhere herein, in some embodiments, the flexibility of an element, layer, or region of the device 100 may be modified by incorporation of one or more additives (e.g., a plasticizer) into the element, layer, or region. In some embodiments, the reservoir 102 may swell at a first rate and the backing element 106 may swell at a second rate, where the first rate and the second rate differ by less than about 50%, in some embodiments less than about 20%, in some embodiments less than about 10%, in some embodiments less than about 5%, and in some embodiments less than about 1%.

### Reservoir

In general, the reservoir 102 comprises an agent to be delivered to a tissue site and it may constitute a single continuous volume of the device 100 or the reservoir 102 may comprise two or more compartments containing one or more agents or other compositions. Typically, for example, the reservoir 102 comprises an agent, a pharmaceutically acceptable carrier, and optionally one or more excipient, and combinations thereof. Exemplary excipients include stabilizers, glidants, bulking agents, anti-adherents, disintegrants, binders, sorbents, preservatives, cryoprotectants, permeation enhancers, hydrating agents, enzyme inhibitors, mucus modifying agents (e.g., mucus drying agents, etc.), pH modifying agents, solubilizers, plasticizers, crystallization inhibitors, bulk filling agents, bioavailability enhancers, and combinations thereof. In some embodiments, the reservoir 102 may include polyethylene glycols, polyethylene oxides, humectants, vegetable oils, medium chain mono, di-, and triglycerides, lecithin, waxes, hydrogenated vegetable oils, colloidal silicon dioxide, polyvinylpyrrolidone (PVP) ("povidone"), celluloses, CARBOPOL® polymers (Lubrizol Advanced Materials, Inc.) (i.e., crosslinked acrylic acid-based polymers), acrylate polymers, pectin, sugars, magnesium sulfate, or other hydrogel forming polymers.

In some cases, the reservoir 102 may include a release-rate control excipient. In some embodiments, the release-rate control excipient may enhance the release rate of the agent as compared to a device 100 without the release-rate control excipient. In some embodiments, the release rate control excipient may retard the release rate of the agent as compared to a device without the release rate control excipient. In some cases, the release-rate control excipient may be a surfactant (e.g., Tween or pyridinium propyl sulfonate). In other embodiments, the release-rate control excipient may be a high ionic strength material (e.g., sodium chloride). Without wishing to be bound by any theory, the release-rate control excipient may increase the rate of agent release by decreasing the affinity of the agent for polymers that may be used to make the reservoir.

In some embodiments, the release-rate control excipient may be any material capable of blocking an agent from binding to a material used to make the reservoir 102. In one non-limiting example, a protein (e.g., bovine serum albumin) that may be capable of binding to a polymer used to make the reservoir 102 may be combined with an agent in an amount sufficient to block the agent from binding to the polymer used to make the reservoir, thus facilitating release of the agent from the device.

In certain embodiments, the release-rate control excipient may be a water-soluble leachable material (e.g., a salt such as sodium chloride, a sugar such as sucrose, and the like). In some embodiments, the leachable material may dissolve when the device 100 is in contact with an aqueous solution, thereby creating channels within the device 100 (e.g., a passageway forming component) and increasing the rate of release of the agent.

In some embodiments, the reservoir 102 may include a release rate-modifying polymer. For example, in one embodiment, the release rate-modifying polymer may be degradable or non-degradable. By way of further example, in one embodiment, the release rate-modifying polymer may be a natural polymer or a synthetic polymer. By way of further example, in one embodiment, natural or synthetic polymers include poly(hydroxy acids), polyanhydrides, polyorthoesters, polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polysiloxanes, polystyrene, polyurethanes, derivatized celluloses, polymers of acrylic acid, methacrylic acid or copolymers or derivatives thereof including esters and acids, and the like. Exemplary poly(hydroxy acids) include poly(lactic acid), poly(glycolic acid), polylactic acid-co-glycolic acid), and the like. Exemplary polyalkylenes include polyethylene and polypropylene, and the like. Exemplary polyalkylene glycols include poly(ethylene glycol), and the like. Exemplary polyalkylene oxides include poly(ethylene oxide), and the like. Exemplary polyalkylene terepthalates include poly(ethylene terephthalate), and the like. Exemplary polyvinyl esters include poly(vinyl acetate), and the like. Exemplary polyvinyl halides include poly(vinyl chloride), and the like. Exemplary derivatized celluloses include alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxyethyl cellulose, cellulose triacetate, cellulose sulfate sodium salt (jointly referred to herein as "synthetic celluloses"), and the like. Exemplary polymers of acrylic acid, methacrylic acid or copolymers or derivatives thereof including esters further include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(lactide-co-caprolactone), and the like (jointly referred to herein as "polyacrylic esters"), and copolymers and blends thereof. Exemplary polymers of acrylic acid, methacrylic acid or copolymers or derivatives thereof including acids further include poly(butyric acid), poly(valeric acid), and copolymers and blends thereof. Examples of degradable release rate-modifying polymers include biodegradable polymers, which include polymers of hydroxy acids such as lactic acid and glycolic acid, and copolymers with PEG, polyanhydrides, poly(ortho)esters, polyurethanes, poly(butyric acid), poly(valeric acid), poly(lactide-co-caprolactone), and blends and copolymers thereof. Examples of non-degradable release rate-modifying polymers include non-biodegradable polymers, which include ethylene vinyl acetate, poly(meth)acrylic acid, polyamides, and copolymers and blends thereof.

In some embodiments, the release of the agent from the reservoir 102 may be controlled by a hydrated state of the reservoir 102 compared to a dry state of the reservoir. For example, in one embodiment, hydration of the reservoir 102 creates a liquid or gel state of the reservoir 102, and creates a pressure that may be utilized to expel the agent from the reservoir 102 through the population of passageways 110 to the intestinal site. Alternatively stated, expulsion of the agent via the pressure increases the agent concentration at the intestinal site compared to expulsion of the agent without the pressure (e.g., passive diffusion from the reservoir with the same agent loading). In one embodiment, the liquid or gel state of the reservoir may include an oil. Exemplary oils may include olive oil or vegetable oil. In another embodiment, the oils may include a viscosity so that the agent may be forced through the mucoadhesive element 104 using the pressure.

In some embodiments, the device 100 may be configured for controlled release of an agent. For example, in one embodiment, a release rate for the agent may be at least 20% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 50% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 80% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 90% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 95% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 99% within 2 hours, as determined by a USP Dissolution Assay 711 with Apparatus 1 and a dissolution medium of 150 mM phosphate buffered saline at pH 6.5. By way of further example, in one embodiment, the release rate for the agent may be at least 99.9% within 2 hours, as determined by a USP Dissolution Assay 711 with Apparatus 1 and a dissolution medium of 150 mM phosphate buffered saline at pH 6.5. In some embodiments, the device 100 may be configured for a burst release of an agent. In some embodiments, the device 100 may be configured to release the agent via convective flow of the agent. In some instances, two or more agents may be located in different regions of the device 100. In some embodiments, the two or more agents may each be different from each other, or at least some of the two or more agents may be the same. Such configurations may be advantageous, for example, for controlling the release rate of an agent. For instance, a first agent in a first element, layer, or region of the reservoir 102 may be configured to release at a first rate, and a second agent in a second element, layer, or region of the reservoir 102 may be configured to release at a second rate, where the first rate and the second rate are different. For example, the first rate may be higher (e.g., a burst release) than the second rate (e.g., a sustained release, or a convective flow). A burst release refers to a much higher release rate during a first period of time as compared to a second period of time. In some embodiments, the burst release may occur during the initial period of drug release (i.e., beginning when the device is placed in an environment in which drug release can occur). In other embodiments, the burst release may occur after a period of sustained release (e.g., zero-order release). An initial burst release may be advantageous, in some embodiments, for rapidly achieving a desired blood concentration of the agent in a subject and thereafter a slower sustained release of the agent may be used to maintain a desired blood concentration of the agent. In some embodiments, the first agent and the second agent may be the same or different. In another embodiment, two or more formulations of one or more agents may be included in the device. The formulations may be contained in separate elements, layers, or regions or may exist as a mixture. In some embodiments, a first formulation may have a first rate of release and the second formulation may have a second rate of release, where the first rate of release is different from the second rate of release.

In some embodiments, absorption of an agent may be improved by a permeation enhancer expelled from the reservoir 102, along with expulsion of the agent from the reservoir 102. In some embodiments, a seal formed by the mucoadhesive element 104 of the device 102 may substantially prevent escape of the permeation enhancer from a privileged region also formed by the mucoadhesive element 104. In some embodiments, the permeation enhancer may be substantially retained in the privileged region after expulsion from the reservoir 102. In some embodiments, expulsion of the permeation enhancer from the reservoir 102 may reduce the viscosity of mucus. In some embodiments, the permeation enhancer may be capable of opening a tight junction between cells (e.g., intestinal cells or epithelial cells). A permeation enhancer may, in some instances, facilitate uptake of an agent into epithelial cells.

Representative classes of permeation enhancers include, but are not limited to, a fatty acid, a medium chain glyceride, a surfactant, a steroidal detergent, an acyl carnitine, lauroyl-DL-carnitine, an alkanoyl choline, an N-acetylated amino acid, esters, salts, bile salts, sodium salts, nitrogen-containing rings, derivatives thereof, and combinations thereof. The permeation enhancer may be anionic, cationic, zwitterionic, or nonionic. Anionic permeation enhancers include, but are not limited to, sodium lauryl sulfate, sodium decyl sulfate, sodium octyl sulfate, N-lauryl sarcosinate, and sodium carparate. Cationic permeation enhancers include, but are not limited to, cetyltrimethyl ammonium bromide, decyltrimethyl ammonium bromide, benzyldimethyldodecyl ammonium chloride, myristyltrimethylammonium chloride, and dodecylpyridinium chloride. Zwitterionic permeation enhancers include, but are not limited to, N-dodecyl-N,N-dimethyl-3-ammonio-1-propanesulfonate, 3-(N,N-dimethylpalmitylammonio)propanesulfonate. Fatty acids include, but are not limited to, butyric, caproic, caprylic, pelargonic, capric, lauric, myristic, palmitic, stearic, arachidic, oleic, linoleic, and linolinic acid, salts thereof, derivatives thereof, and combinations thereof. In some embodiments, a fatty acid may be modified as an ester, for example, a glyceride, a monoglyceride, a diglyceride, or a triglyceride. Bile acids or salts including conjugated or unconjugated bile acid permeation enhancers include, but are not limited to, cholate, deoxycholate, tauro-cholate, glycocholate, taurodexycholate, ursodeoxycholate, tauroursodeoxycholate, chenodeoxycholate, derivates thereof, salts thereof, and combinations thereof. In some embodiments, permeation enhancers include a metal chelator, such as EDTA or EGTA, a surfactant such as sodium dodecyl sulfate, polyethylene ethers or esters, polyethylene glycol-12 lauryl ether, salicylate polysorbate 80, nonylphenoxypolyoxyethylene, dioctyl sodium sulfosuccinate, saponin, palmitoyl carnitine, lauroyl-l-carnitine, dodecyl maltoside, acyl carnitines, alkanoyl cjolline, and combinations thereof. Other permeation enhancers include, but are not limited to, 3-nitrobenzoate, zoonula occulden toxin, fatty acid ester of lactic acid salts, glycyrrhizic acid salt, hydroxyl beta-cyclodextrin, N-acetylated amino acids such as sodium N-[8-(2-hydroxybenzoyl)amino]caprylate and chitosan, micelle forming agents, passageway forming agents, agents that modify the micelle forming agent, agents that modify the passageway forming agents, salts thereof, derivatives thereof, and combinations thereof. In some embodiments, micelle forming agents include bile salts. In some embodiments, passageway forming agents include antimicrobial peptides. In some embodiments, agents that modify the micelle forming agents include agents that change the critical micelle concentration of the micelle forming agents. An exemplary permeation enhancer is 1% by weight 3-(N,N-dimethylpalmitylammonio)propanesulfonate. Permeation enhancers are also described in patent application publication US 2013/0274352,

In some embodiments, a permeation enhancer may be included in a device 100 (e.g., a wafer) at a concentration of between about 0.001% to about 10% by weight, between about 0.001 % to about 5% by weight, between about 0.001 % to about 1% by weight, between about 0.1% to about 5% by weight, between about 0.5% to about 2% by weight, between about 0.01% to about 10% by weight, between about 0.1 % to about 10% by weight, or between about 1% to about 10% by weight. In other embodiments, a permeation enhancer may be included in a device (e.g., a wafer) at a concentration of greater than 0.001 % by weight, greater than 0.01 % by weight, greater than 0.05% by weight, greater than 0.1% by weight, greater than 0.5% by weight, greater than 1% by weight, greater than 2% by weight, greater than 5% by weight, or greater than 10% by weight.

In certain embodiments, the agent may be released in an essentially linear fashion. For example, in one embodiment, the release may be essentially zero-order. In some embodiments, the release may be non-linear. In some embodiments, the release may be non-linear for a first period of time and essentially linear for a second period of time.

In some embodiments, the reservoir 102 may include a plurality of microparticles and/or nanoparticles for controlled release of the agent. The particles may, in some embodiments, be formed from one or more polymers, such as those described herein. In some embodiments, the particles may be dispersed throughout the reservoir 102. In other embodiments, the particles may be located in a particular region of the reservoir 102. By way of further example, peptide conjugates include, without limitation, cell penetrating peptides such as TAT, PTD, polyarginine, polylysine, and the like, and combinations thereof. By way of further example, in one embodiment, fusogenic peptide conjugates may include, without limitation, KALA, melittin, and the like, and combinations thereof.

In some embodiments, the reservoir 102 may include a release element that modulates the release of an agent from the device. The release element may, in some embodiments, be disposed on the mucoadhesive element 104. In some embodiments, the release element may be polymeric. In some instances, the release element may be degradable or non-degradable. In certain embodiments, the release element may have passageways, as described elsewhere herein, through which the agent can elute.

### Other Elements

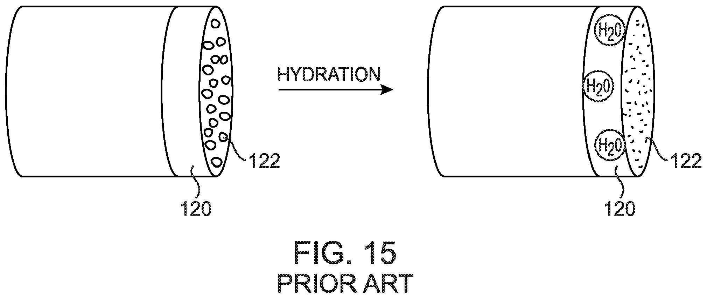

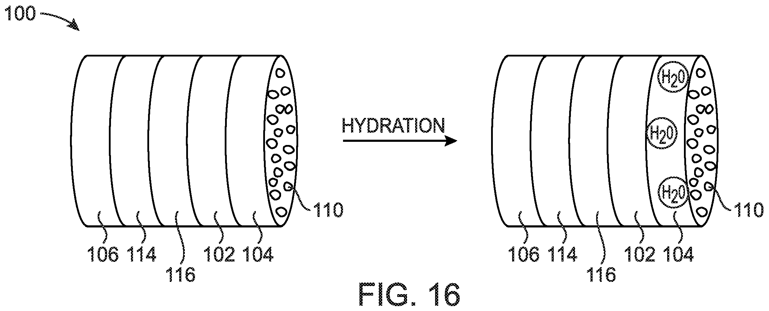

In general, the device 100 may include a plurality of elements, layers, compartments, sub-compartments, and the like, and combinations thereof, for the delivery of an agent to a tissue site. The device 100 may be adapted for the directed delivery of an agent wherein the agent within the device may be further protected from degradation in the gastrointestinal tract of an animal or a human. For example, the device 100 includes a backing element 106 for excluding the premature escape of an agent to the intestinal lumen. Alternatively stated, the backing element 106 may assist in the directed delivery of the agent promptly to a tissue site. By way of further example, the device 100 further includes a reservoir 102 where the agent may be disposed within the reservoir. The agent disposed within the reservoir 102 may include more than one agent. Further, more than one agent may include different agents. By way of further example, the device 100 further includes a mucoadhesive element 104 for binding of the device 100 to a tissue site. By way of further example, the device 100 further includes passageways 110 within the mucoadhesive element 104 that the agent passes through, from the reservoir 102, en route to the tissue site. The passageways 110 are advantageous in that the passageways 110 remain open when the device 100 is administered, and continue to remain open for complete delivery of the agent. More specifically, prior art mucoadhesives 120 contain pores 122 that close upon hydration, as shown in FIG. 15. Conversely, the inventive subject matter of the instant disclosure pertains to passageways 110 that remain open even after the mucoadhesive element (M) becomes hydrated, as shown in FIG. 16.

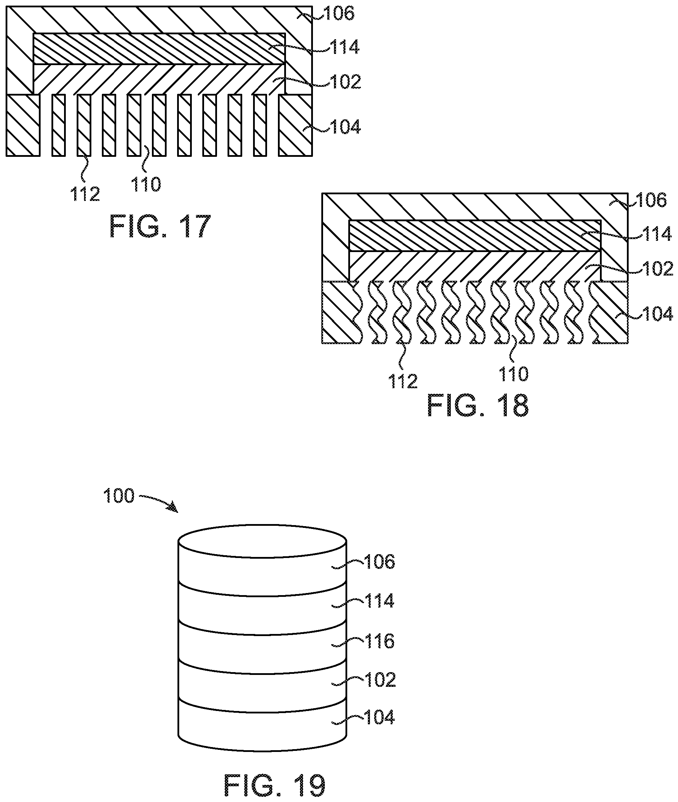

By way of further example, the device 100 may include other elements. For example, as shown in FIGS. 17 and 18, the device may include a push element 114 for the "push" or active expulsion of the agent from the reservoir 102 through the mucoadhesive element 104. By way of further example, the push element 114 may provide for a controlled delivery profile for the agent. By way of further example, as shown in FIG. 19, in some embodiments, the device may include an optional barrier element 116. The barrier element may reside between any of the aforementioned elements. For example, the barrier element 116 may be disposed between the backing element 106 and the reservoir 102. The barrier element 116 may prevent one element from co-mixing with another element. By way of further example, in some embodiments, the device 100 may include an optional drying element. The drying element may coacervate or condense mucus. For example, in one embodiment, loose mucus or intestinal fluids may be condensed onto or into the device via the drying element in order to reduce the amount of liquid for adhesion at the intestinal site. By way of further example, in some embodiments, the device 100 may include an optional sacrificial element. The sacrificial element may prevent or reduce release of the agent before the device 100 reaches the tissue site. By way of further example, in some embodiments, the device 100 may include an optional enteric element. The enteric element may protect the device from degradation in the stomach of an animal or a human, as would be appreciated by those of skill in the art. By way of further example, in some embodiments, the device 100 may include an optional mechanical element wherein the mechanical element may be included within the mucoadhesive element to assist in the binding of the device to the tissue site.

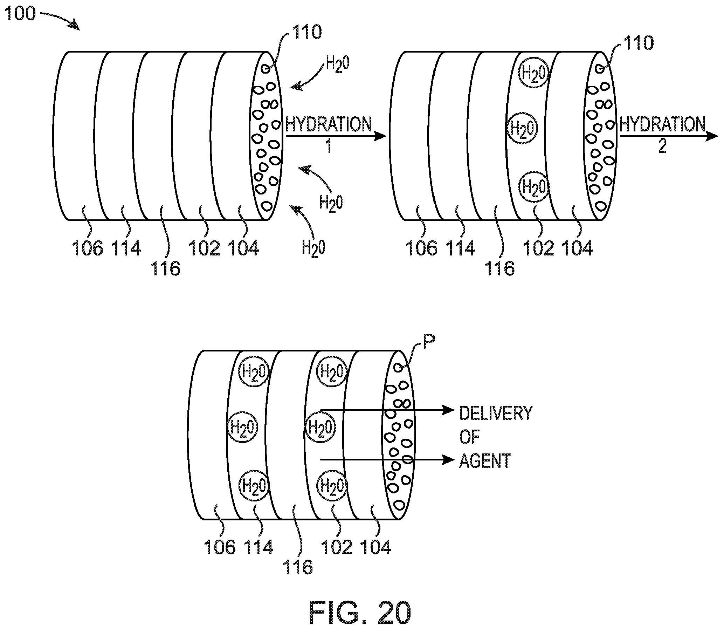

In one embodiment, the device 100 may further include a push element 114. In some embodiments, the push element 114 may be located above the reservoir 102 which may be above the mucoadhesive element 104 having passageways 110, as shown in FIGS. 17 and 18. The push element 114 may operate via an osmotic force to push, eject, or induce convective flow of the agent from the reservoir through the passageways to the intestinal site. The generation of the osmotic force may be via an osmotic agent or osmagent within the push layer. As shown in FIG. 20, the osmagent may be used to draw a solvent (e.g., water) into the push element 114 thereby expanding the volume of the push element 114 (e.g., relative to the reservoir 102, to accommodate the water) thereby generating the force needed to expel the agent from the reservoir 102. In one embodiment, the osmagent may include any suitable material that is capable of being hydrated (e.g., a desiccant). In another embodiment, the push element 114 may include any suitable material that is capable of generating a gas. For example, in one embodiment, an osmagent capable of being hydrated may include water-soluble salts, carbohydrates, small molecules, amino acids, water-soluble hydrogel forming polymers, and combinations thereof. Exemplary water-soluble salts may include, without limitation, magnesium chloride, magnesium sulfate, lithium chloride, sodium chloride, potassium chloride, lithium sulfate, sodium sulfate, potassium sulfate, sodium hydrogen phosphate, potassium hydrogen phosphate, sodium acetate, potassium acetate, magnesium succinate, sodium benzoate, sodium citrate, sodium ascorbate, and the like, and combinations thereof. Exemplary carbohydrates may include sugars such as arabinose, ribose, xylose, glucose, fructose, galactose, mannose, sucrose, maltose, lactose, raffinose, and the like, and combinations thereof. Exemplary amino acids may include glycine, leucine, alanine, methionine, and the like, and combinations thereof. Exemplary water-soluble hydrogel forming polymers may include sodium carboxy methylcellulose, hydroxypropyl methylcellulose (HPMC), hydroxyethyl methylcellulose, crosslinked PVP, polyethylene oxide, carbopols, polyacrylamindes, and the like, and combinations thereof. In another embodiment, the push element 114 including the osmagent capable of being hydrated may have a swelling ratio of a hydrated push element to a non-hydrated push element. The swelling ratio may be defined by cryo-SEM and scanning electron microscopy. For example, the swelling ratio of the push element 114 may be measured by an Average Wet Thickness of the push element compared to an Average Dry Thickness of the push element. By way of further example, the Wet Thickness of the push element 114 may be measured by cryo-SEM following 30 minutes of hydration.

In one exemplary embodiment, the cryo-SEM experiments may be performed using typically a very small volume of push element 114 (5-10 µl). A very small volume of push element 114 may be as large as one should consider freezing because the larger the volume, the slower the freezing, a condition that would result in structural damage due to hexagonal ice formation. Balzers gold planchets (BU012 130T) may be used for cryoimmobilization of hydrated push elements. Once the push element 114 has been frozen by plunge or high-pressure freezing (HPF) methods, store the samples in liquid nitrogen prior to loading onto the cryostage. All handling tools must be chilled prior to touching the planchet. the sample surface may be fractured by lightly scraping the planchet surface under liquid nitrogen with a pre-chilled blade. A deep surface fracture should be avoided as this surface is the best part of the sample. An in-lens DS-130 FESEM (Topcon) having a TEM-like cryostage (Gatan CT-3500) equipped with dual protective shutters may be used. Next, best practices include dry nitrogen venting the dedicated high vacuum sputter coater chamber prior to rapid transfer from the liquid nitrogen work station. The system should then be evacuated to 10⁻⁷ torr to remove all O₂. Then, by slowly raising the specimen temperature to between -105 °C and -120 °C, ice can be sublimed out of the push element. The rate of sublimation (etching) may be very dependent on vacuum. Balzers provides a guide for 10⁻⁷ torr vacuum systems such that at -115 °C the sublimation rate is ∼1Å/ sec and ~100 Å/ sec at -105 °C. In this sample there is the *bulk* water, the *tightly bound* water, or the hydration shell in contact with the solid phase; and *loosely bound* water that are molecules local to the tightly bound water. Since the hydration properties of each sample vary, and differences occur within different regions of the same sample, etching times should be experimentally derived. Care should be taken when etching because there may be a risk of over-etching the sample and stripping away the hydration shell, thus freeze drying the sample solid phase, thereby leading to artifacts. After the etching time has been completed, the specimen is returned to less than -180 °C and sputter coated with 1-2 nm chromium (Cr). The stage should then be transferred into the microscope and allowed to equilibrate for 30 minutes at the desired imaging temperature. Rapid digital recordings should be made at 5-30 keV in the LTSEM. Attention to preparation details will facilitate high-quality cryo-SEM images.

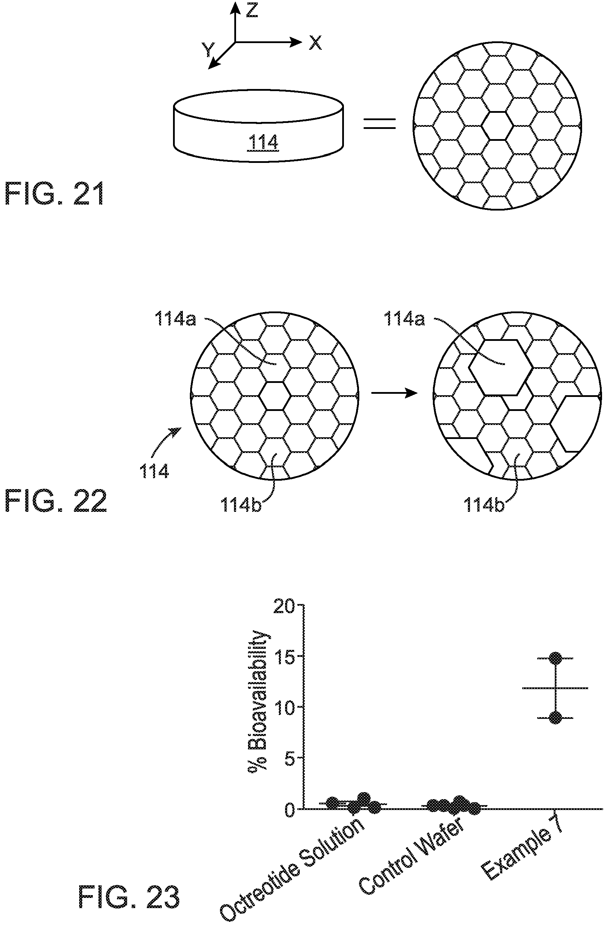

The Dry Thickness of the push element 114 may be measured by scanning electron microscopy. In some embodiments, for example, the swelling ratio may be at least 1.5. By way of further example, in one embodiment, the swelling ratio may be at least 2. By way of further example, in one embodiment, the swelling ratio may be at least 3. By way of further example, in one embodiment, the swelling ratio may be at least 4. By way of further example, in one embodiment, the swelling ratio may be at least 5. In some embodiments, the expansion of the push element may be adjusted. For example, in one embodiment, adjustment of the push element expansion may include increasing the concentration of the osmagent within the push element 114 thereby increasing the release rate of the agent from the reservoir 102. By way of further example, in one embodiment, adjustment of the push element expansion may include increasing the swelling ratio of the push layer 114 relative to the reservoir 102thereby increasing the release rate of the agent from the reservoir. By way of further example, in one embodiment, adjustment of the push element expansion may include adjusting the water permeability of the backing element 106 (as described elsewhere herein) thereby increasing the release rate of the agent from the reservoir 102. In some embodiments, the push element 114 having the osmagent capable of being hydrated may be subdivided into one or more independent push element compartments, for example, using a barrier element 116 (as described elsewhere herein), each having the osmagent. In one embodiment, the push element compartment may be shaped as a polygon. By way of further example, in one embodiment, the polygon shape may include a honeycomb shape, as shown in FIG. 21. The multiple independent push element compartments may allow for multiple independent ejections of the agent from the reservoir through the passageways to the intestinal site. For example, in one embodiment, a first push element compartment 114a may swell upon hydration and eject the agent before a second push element compartment 114b swells, as shown in FIG. 22.

In another embodiment, a push element 114 capable of generating a gas may include an acid and a solid base. For example, hydration of the acid and solid base allows for a reaction that generates a gas (e.g., carbon dioxide). The generation of the gas provides the pressure needed for the expulsion of the agent through the passageways 110 to the intestinal site. Exemplary acids may include stomach acid (i.e., hydrochloric acid), solid organic acids, solid inorganic acids, and the like. In one embodiment, solid organic acids may include carboxylic acids and phenols. For example, in one embodiment, carboxylic acids may include citric acid, tartaric acid, maleic acid, and the like, and combinations thereof. By way of further example, in one embodiment, phenols may include polyphenols such as tannic acid, and the like, and combinations thereof. In one embodiment, solid inorganic acids may include phosphoric acid, boric acid, and the like, and combinations thereof. Exemplary solid bases may include carbonate salts. For example, in one embodiment, a carbonate salt may include calcium carbonate, potassium carbonate, sodium bicarbonate, and the like, and combinations thereof. In one embodiment, the push element 114 capable of generating a gas may have an onset of hydration time. In another embodiment, the push element 114 capable of generating a gas may have a volume of gas generated. In yet another embodiment, the push element 114 capable of generating a gas may have a total release time.

In some embodiments, the expansion of the push element 114 via a gas may be adjusted. For example, in one embodiment, adjustment of the push element gas expansion may include increasing the concentration of an osmagent within the push element 114 thereby increasing the release rate of the agent from the reservoir 102. By way of further example, in one embodiment, adjustment of the push element gas expansion may include increasing the swelling ratio of the of the push layer 114 relative to the reservoir 102 thereby increasing the release rate of the agent from the reservoir 102 (i.e., by increasing the pressure of the gas released). By way of further example, in one embodiment, adjustment of the push element gas expansion may include adjusting the water permeability of the backing element 106 (as described elsewhere herein) thereby increasing the release rate of the agent from the reservoir 102 (i.e., by increasing the pressure of the gas released). Alternatively stated, increasing the pressure of the gas allows for increased permeability of the agent through the population of passageways 110 to the intestinal site. By way of further example, in one embodiment, the gas generated in the push element 114 may be prefilled into a pressurized capsule and subsequently released. In some embodiments, the push element 114 capable of generating a gas may be subdivided into one or more independent push element compartments, for example, using a barrier element 116 (as described herein), each having the capability of generating the gas. In one embodiment, the push element compartment may be shaped as a polygon. By way of further example, in one embodiment, the polygon may include a honeycomb shape, as shown in FIG. 21. The multiple independent push element compartments may allow for multiple independent ejections of the agent from the reservoir through the passageways to the intestinal site. For example, in one embodiment, a first push element compartment 114a may swell upon hydration and eject the agent before a second push element compartment 114b swells, as shown in FIG. 22. In some embodiments, when the expulsion of the agent - after adhesion to a tissue - is driven by osmotic hydration or gas-driven expansion of a push element, the agent may flow out convectively. For example, in one embodiment, a thicker enteric element around the push element 114 may effectively delay hydration of the push element until the reservoir is fully hydrated. Stated alternatively, convective flow of the agent begins after hydration of the push element which is after full hydration of the reservoir 102. Osmotic hydration or gas-driven expansion of the push element, as measured by cryo-SEM, may deliver, for example in one embodiment, greater than 50% of the agent before the mucoadhesive element releases from the tissue. By way of further example, in one embodiment, osmotic hydration or gas-driven expansion of the push element 114, as measured by cryo-SEM, may deliver greater than 50% of the agent in the first 1 hour after placement of the device in saline on top of a piece of tissue.

In some embodiments, the device 100 may optionally include a drying element for coacervating or condensing mucus. For example, in one embodiment, loose mucus or intestinal fluids may be condensed onto or into the device 100 via the drying element in order to reduce the amount of liquid for adhesion at the intestinal site. In some embodiments, coacervation may include the use of charged based polymers. For example, in one embodiment, charged based polymers may include cationic polymers. Exemplary cationic polymers may include chitosan, guar hydroxypropyltrimonium chloride, quaternized hydroxyethylcelluloses, diethylaminoethyl dextran, polylysine, hydroxyethylated polyethylenimine, and the like, and combinations thereof. Exemplary quaternized hydroxyethylcelluloses may include polyquaternium-10, polyquaternium-24, polyquaternium-67, and the like, and combinations thereof.

In some embodiments, the device 100 may optionally include a sacrificial element that essentially prevents or substantially reduces release of the agent. For example, in some embodiments, the sacrificial element may coat the opposing surface 112 of the mucoadhesive element 104. In some embodiments, the sacrificial element may substantially coat the entire device 100. The sacrificial element may include any suitable material that can prevent or reduce release of the agent when such properties are desirable and can allow or increase release of the agent when such properties are desirable. In some embodiments, the release-altering properties of the sacrificial element decrease as the sacrificial element dissolves or degrades. Thus, in some embodiments, the sacrificial element may be any suitable material that performs as described above. In some embodiments, the sacrificial element may be a polymer, for example, a degradable or non-degradable polymer, as described elsewhere herein.

In some embodiments, a system may include a plurality of devices 100 encapsulated in a containment vehicle. For instance, the plurality of devices 100 may be encapsulated in a capsule, a caplet, a gelcap, or a tablet. The containment vehicle may be configured, in some embodiments, to dissolve in certain regions of a subject (e.g., the small intestine or the large intestine) and/or under certain conditions (e.g., within certain pH ranges). For example, the containment vehicle may be enteric coated. An enteric coating may be any suitable coating that allows the containment vehicle to release the devices in the small intestine. In some cases, an enteric coating may dissolve preferentially in the small intestine as compared to the stomach. In other embodiments, the enteric coating may hydrolyze preferentially in the small intestine as compared to the stomach. Non-limiting examples of materials used as enteric coatings include methyl acrylate-methacrylic acid copolymers, cellulose acetate succinate, hydroxy propyl methyl cellulose phthalate, hydroxy propyl methyl cellulose acetate succinate (i.e., hypromellose acetate succinate), polyvinyl acetate phthalate (PVAP), methyl methacrylate-methacrylic acid copolymers, and sodium alginate, and stearic acid. Other examples and embodiments are discussed in more detail elsewhere herein. In some embodiments, the device may be coated with one or more enteric coatings. For example, in one embodiment, a first enteric coating may be different than a second enteric coating. In some embodiments, each of the enteric coatings may have a front surface, a back surface, the front surface opposing the back surface; an upper surface and a bottom surface, the upper surface opposing the bottom surface; and a left lateral surface and a right lateral surface, the left lateral surface opposing the right lateral surface. In some embodiments, the capsule may contain, in addition to the device 100, permeation enhancers (as described elsewhere herein), enzyme inhibitors (e.g., protease inhibitors), mucus modifying agents, excipients, and the like, and combinations thereof. In some embodiments, a portion or all of the capsule may include the mucoahesive element to allow for capsule degradation and agent release near the intestinal adhesion site. An enteric coating may also be applied to the devices 100 described herein (e.g., a wafer), regardless of whether the containment vehicle is enteric coated or not. For example, if the containment vehicle is not enteric coated, and the device(s) 100 within the containment vehicle is (are) enteric coated, the containment vehicle may preferentially dissolve in the stomach, allowing the device(s) 100 within the containment vehicle to be released; and the enteric coating of the device(s) 100 allows the device(s) 100 to dissolve preferentially in the small intestine as compared to the stomach. For example, in one embodiment, the enteric coating may be soluble at a pH greater than 5.5. By way of further example, in one embodiment, the enteric coating may be soluble at a pH greater than 6.5. By way of further example, in one embodiment, the enteric coating may be soluble at a pH within a range of about 5.6 to 6. By way of further example, in one embodiment, the enteric coating may be soluble at a pH within a range of about 5.6 to 6.5 to 7. In some embodiments, the dissolution time at an intestinal pH may be controlled or adjusted by the composition of the enteric coating. In some embodiments, the dissolution time at an intestinal pH may be controlled or adjusted by the thickness of the enteric coating. In some embodiments, the composition and the thickness of the enteric coating may be varied to expose different areas of the device at different times. For example, in one embodiment, the device or containment vehicle may be non-isotropically coated to allow for a particular orientation of the device 100 upon release of the agent in the intestine. By way of further example, in one embodiment, the backing element 106 may be coated with a thicker enteric coating than the mucoadhesive element 104 to allow for the device 100 to adhere to the intestinal site before release of the agent via hydration of the reservoir 102 and/or the push element 114.

In some embodiments, the device 100 may optionally include a barrier element 116. For example, the barrier element 116 may reside between any two elements or compartments, as described herein. By way of further example, in one embodiment, the barrier element 116 may be disposed between the reservoir 102 and the push element 114. By way of further example, in one embodiment, the barrier element 116 may be disposed between the backing element 106 and the push element 114. By way of further example, the barrier element 116 may be disposed between or define the push element compartment, as described herein. Suitable materials for forming the barrier element 116 may include polyethylene, polystyrene, ethylene-vinyl acetate copolymers, polycaprolactone and Hytrel™ polyester elastomers (i.e., as supplied by Du Pont), cellulose acetate, cellulose acetate pseudolatex (as described in US 5,024,842, cellulose acetate propionate, cellulose acetate butyrate, ethyl cellulose, ethyl cellulose pseudolatex (such as Surelease™ as supplied by 10 Colorcon, West Point, Pa. or Aquacoat™ as supplied by FMC Corporation, Philadelphia, Pa.), nitrocellulose, polylactic acid, poly-glycolic acid, polylactide glycolide copolymers, collagen, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters (such as Eudragit™ supplied by RohmPharma, Darmstaat, Germany), polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyalkoxysilanes, polydimethyl siloxane, polyethylene glycol-silicone elastomers, electromagnetic irradiation crosslinked acrylics, silicones, or polyesters, thermally crosslinked acrylics, silicones, or polyesters, butadienestyrene rubber, and the like, and combinations thereof.

In some embodiments, the barrier element 116 may include cellulose acetate, copolymers of acrylic acid and methacrylic acid esters, copolymers of methylmethacrylate and ethylacrylate, and latex of acrylate esters. Preferred copolymers may include poly(butyl methacrylate), (2-dimethylaminoethyl)methacrylate, methyl methacrylate) 1:2:1, 150,000, sold under the trademark EUDRAGIT E; poly (ethyl acrylate, methyl methacrylate) 2:1, 800,000, sold under the trademark EUDRAGIT NE 30 D; poly(methacrylic acid, methyl methacrylate) 1:1, 135,000, sold under the trademark EUDRAGIT L; poly(methacrylic acid, ethyl acrylate) 1:1, 250,000, sold under the trademark EUDRAGIT L; poly (methacrylic acid, methyl methacrylate) 1:2, 135,000, sold under the trademark EUDRAGIT S; poly(ethyl acrylate, methyl methacrylate, trimethylammonioethyl methacrylate chloride) 1:2:0.2, 150,000, sold under the trademark EUDRAGIT RL; poly(ethyl acrylate, methyl methacrylate, trimethylammonioethyl methacrylate chloride) 1:2:0.1, 150,000, sold as EUDRAGIT RS, and the like, and combinations thereof; wherein the ratio x:y:z indicates the molar proportions of the monomer units, and the six-digit number is the number average molecular weight of the polymer.

In some embodiments, the barrier element 116 may include cellulose acetate containing plasticizers, such as acetyl tributyl citrate, and ethylacrylate methylmethylacrylate copolymers such as Eudragit NE, and the like and combinations thereof.

In some embodiments, the barrier layer 116 may further include a plasticizer for making the barrier element deformable. For example, in one embodiment, the barrier element 116 may be deformable to accommodate the force exerted from the push element 114, as described herein. By way of further example, in one embodiment, the deformation of the barrier element 116 may include contraction or expansion of the barrier element 116, as needed, in association with expulsion of the agent through the population of passageways to the intestinal site. Exemplary plasticizers may include polyhydric alcohols, triacetin, polyethylene glycol, glycerol, propylene glycol, acetate esters, glycerol triacetate, triethyl citrate, acetyl triethyl citrate, glycerides, acetylated monoglycerides, oils, mineral oil, castor oil, and the like, and combinations thereof. In some embodiments, the plasticizers may be blended into the barrier element. For example, in one embodiment, the plasticizers may include 10-50 weight percent based on the weight of the barrier element 116.

Further still, in general, the device 100 may be designed with the elements organized in a plurality of configurations. For example, in some embodiments, the device 100 may have elements that are adapted to encapsulate or cover other elements. By way of further example, in one embodiment, the reservoir 102 may be organized on top of the mucoadhesive element 104, and the reservoir 102 may be covered with the backing element 106. In this embodiment, the backing element 106 may mitigate the loss of the reservoir contents to the intestinal lumen, while the mucoadhesive element 104 allows for the delivery of the reservoir contents after adhering to a tissue site. By way of further example, in one embodiment, the reservoir 102 may reside on top of the mucoadhesive element 104 and the push element 114 may reside between the reservoir 102 and the backing element 106. In this embodiment, the backing element 106 may mitigate the loss of the reservoir contents to the intestinal lumen, the push element 114 may enhance (i.e., push) the delivery of the reservoir contents (e.g., an agent(s)) to the tissue site, all while the mucoadhesive element 104 allows for the delivery of the reservoir contents after adhering to a tissue site. By way of further example, in some embodiments, the push element 114 may be divided into one or more sub-push elements separated via one or more barrier elements 116. In this embodiment, the backing element 106 may mitigate the loss of the reservoir contents to the intestinal lumen, the push element may enhance (i.e., push) the delivery of the reservoir contents (e.g., an agent) to the tissue site in a controlled and step-wise manner via the sub-push elements, all while the mucoadhesive element 104 allows for the delivery of the reservoir contents after adhering to a tissue site. By way of further example, in some embodiments, a drying element may cover the mucoadhesive element 104. In this embodiment, the drying element may improve adhesion of the device 100 via the mucoadhesive element by coacervating or condensing mucus. Other configurations will be apparent to those of skill in the art without departing from the scope of the inventive subject matter.

Yet further still, in general, the device 100 may be configured such that the device 100 does not substantially adhere to (i.e., aggregate with) one or more other devices. For example, in one embodiment, the device 100 may include an anti-adhesion agent that substantially reduces the adhesion of one device for another. In some embodiments, the anti-adhesion agent may be an element or layer of the device 100. In some cases, the layer may at least partially coat the mucoadhesive element 104. In some embodiments, the layer may substantially coat the mucoadhesive element 104. In certain embodiments, the layer may coat the entire device 100. In some embodiments, the anti-adhesive layer may dissolve or degrade over a short period of time to allow the devices to drift away from each other. For example, in one embodiment, the anti-adhesion layer may be configured to dissolve or degrade over a period of between about 1 minute and about 180 minutes, in some embodiments between about 1 minute and about 120 minutes, in some embodiments between about 1 minute and about 60 minutes, in some embodiments between about 1 minute and about 30 minutes, in some embodiments between about 10 minutes and about 120 minutes, in some embodiments between about 20 minutes and about 120 minutes, and in some embodiments between about 30 minutes and about 120 minutes. In some embodiments, the anti-adhesive agent may be prepared from, for example, sugars, polymers, proteins, or other molecules. In a non-limiting example, the anti-adhesion agent may be a polyalkylene glycol (e.g., polyethylene glycol), silica, and/or magnesium stearate. In certain embodiments, the anti-adhesive layer may include a dispersal agent or a disintegrant. For example, in one embodiment, the disintegrant may be an expandable polymer, crosslinked polyvinylpyrrolidone (crospovidone), crosslinked sodium carboxymethyl cellulose (croscarmellose sodium), and sodium starch glycolate.

In some embodiments, the mucoadhesive element 104 may include mechanical elements to enhance adhesion to the intestinal site. For example, in one embodiment, mechanical elements may include physical protrusions. By way of further example, in one embodiment, physical protrusions may include hooks, needles, suction cups, and the like.

In some embodiments, the mucoadhesive element 104 may also include mucus modifying agents. For example, in some embodiments, the mucus modifying agent may include N-acetyl cysteine, thioglycolates, small molecule reducing agents, guaifenesin, capsaicin, and the like, and combinations thereof.

In some embodiments, the mucoadhesive element 104 may also include mucus drying agents. For example, in some embodiments, the mucus drying agent may include silica powder, silica gel, calcium chloride, calcium sulfate, magnesium sulfate, cobalt chloride, and the like, and combinations thereof.

In some embodiments, the mucoadhesive element104 may also include pH modifying agents. pH modifying agents are well known to those of skill in the art, and include acids, bases, salts, buffers, and the like, and combinations thereof.

### Device

In general, and without being bound to any particular theory, the elements above may be substantially cylindrical, spherical, or polygonal. For example, in a non-limiting embodiment, the element may be rectangular block-shaped having six surfaces. For example, in one embodiment, the backing element 106 includes a front and back surfaces, the front surface being opposite the back surface; an upper surface and a bottom surface, the upper surface being opposite the bottom surface; and a left and right lateral surfaces, the left lateral surface being opposite the right lateral surface. By way of further example, in one embodiment, the reservoir 102 also includes a front and back surfaces, the front surface being opposite the back surface; an upper surface and a bottom surface, the upper surface being opposite the bottom surface; and a left and right lateral surfaces, the left lateral surface being opposite the right lateral surface. By way of further example, in one embodiment, the mucoadhesive element 104 also includes a front and back surface, the front surface being opposite the back surface; an upper surface and an opposing surface 112, the upper surface being opposite the opposing surface 112; a left and right lateral surfaces, the left lateral surface being opposite the right lateral surface; and wherein the opposing surface 112 adheres to and opposes a tissue site. By way of further example, in one embodiment, any one of the surfaces of a first element (e.g., arbitrarily, the bottom surface of the reservoir) may be in direct contact with any one of the surfaces of a second element (e.g., arbitrarily, the upper surface of the mucoadhesive element 104).

In general, the devices 100 described herein may have any suitable dimensions. For example, in one embodiment, the device has a total surface area of at least about 0.1 cm². In general, however, the devices of the present disclosure will have a total surface area of no more than about 10 cm². Thus, for example, in one embodiment the device has a total surface area in the range of 0.1 to 10 cm², 0.1 to 5 cm², 0.5 to 10 cm², 0.5 to 5 cm², 0.5 to 2 cm², or 1 to 2 cm².

In one embodiment, the opposing surface 112 of the mucoadhesive element 104 has a surface area of at least about 0.05 cm². Typically, however, the opposing surface 112 will have a surface area of less than about 3 cm². Thus, for example, in one embodiment the opposing surface 112 has a surface area of about 0.05 to about 3 cm². By way of further example, in one embodiment the opposing surface 112 has a surface area of about 0.2 to about 2 cm². By way of further example, in one embodiment the opposing surface 112 has a surface area of about 0.5 to about 1 cm².

Additionally, the opposing surface 112 may comprise a significant fraction of the total surface area of the device 100. Thus, for example, in one embodiment the opposing surface 112 comprises at least about 20% of the total surface area of the device 100. By way of further example, in one embodiment the opposing surface 112 comprises at least about 30% of the total surface area of the device 100. By way of further example, in one embodiment the opposing surface 112 comprises at least about 40% of the total surface area of the device 100. By way of further example, in one embodiment the opposing surface 112 comprises at least about 45% of the total surface area of the device 100. By way of further example, in one embodiment the opposing surface 112 comprises at least about 50% of the total surface area of the device 100. By way of further example, in one embodiment the opposing surface 112 comprises at least about 60% of the total surface area of the device 100. By way of further example, in one embodiment the opposing surface 112 comprises at least about 70% of the total surface area of the device 100. By way of further example, in one embodiment the opposing surface 112 comprises at least about 80% of the total surface area of the device 100. By way of further example, in one embodiment the opposing surface 112 comprises at least about 90% of the total surface area of the device 100. By way of further example, in one embodiment the opposing surface 112 comprises at least about 95% of the total surface area of the device 100. By way of further example, in one embodiment the opposing surface 112 comprises at least about 99% of the total surface area of the device 100.

Further, in general, in some embodiments, any of the elements described herein may have any suitable thickness. The thickness of an element may impart improved device stability. For example, in one embodiment, the thickness of an element may prevent the device from breaking apart. By way of further example, in one embodiment, the thickness of the mucoadhesive element 104 may improve the binding to, and the time bound to, a tissue site and the subsequent delivery of the agent. Further, in general, the thickness of an element may be approximately uniform. Further still, in general, the thickness of an element may be non-uniform. For example, in one embodiment, a non-uniform thickness may be measured by a maximum thickness of an element minus a minimum thickness of the element divided by the average thickness of an element. For example, in one embodiment, the non-uniform thickness may be 0. By way of further example, in one embodiment, the non-uniform thickness may be 0.01. By way of further example, in one embodiment, the non-uniform thickness may be 0.1. By way of further example, in one embodiment, the non-uniform thickness may be 1. By way of further example, in one embodiment, the non-uniform thickness may be 2. A uniform thickness of an element may be measured by the shortest distance from one surface of the element to another surface of the element, as described herein. For example, in one embodiment, the backing element 106 may have a thickness measured from the bottom surface to the upper surface; or from the left lateral surface to the right lateral surface. By way of further example, in one embodiment, the mucoadhesive element 104 may have a thickness measured from the Opposing Surface to the upper surface; or from the left lateral surface to the right lateral surface; or from the front surface to the back surface. For example, in some embodiments, an element or layer may be less than about 10 mm thick. By way of further example, in some embodiments less than about 5 mm thick. By way of further example, in some embodiments, less than about 1 mm thick. By way of further example, in some embodiments, less than about 500 microns thick. By way of further example, in some embodiments, less than about 200 microns thick. By way of further example, in some embodiments, less than about 100 microns thick. By way of further example, in some embodiments, less than about 50 microns thick. By way of further example, in some embodiments, less than about 20 microns thick. By way of further example, in some embodiments, less than about 10 microns thick. By way of further example, in some embodiments, less than about 5 microns thick. By way of further example, in some embodiments, less than about 2 microns thick. By way of further example, in some embodiments, less than about 1 micron thick. By way of further example, in some embodiments, less than about 500 nm thick. By way of further example, in some embodiments, less than about 200 nm thick. By way of further example, in some embodiments, less than about 100 nm thick. By way of further example, in some embodiments less than about 50 nm thick. In certain embodiments, an element or layer may be between about 50 nm thick and about 10 mm thick. For example, in some embodiments, between about 50 nm thick and about 1 mm thick. By way of further example, in some embodiments, between about 500 nm thick and about 10 mm thick. By way of further example, in some embodiments, between about 500 nm thick and about 1 mm thick. By way of further example, in some embodiments, between about 1 micron thick and about 1 mm thick. By way of further example, in some embodiments, between about 1 micron thick and about 100 microns thick. By way of further example, in some embodiments, between about 1 micron thick and about 10 microns thick. By way of further example, in some embodiments, between about 10 microns thick and about 1 mm thick. By way of further example, in some embodiments, between about 50 nm thick and about 10 microns thick. In some embodiments, an element or layer may be at least about 1 micron thick. For example, in some embodiments, at least about 10 microns thick. By way of further example, in some embodiments, at least about 100 microns thick. By way of further example, in some embodiments, at least about 1 mm thick. By way of further example, in some embodiments, at least about 10 mm thick.

Further still, in general, in some embodiments, the device 100 may be sufficiently flexible such that the device 100 may be capable of flexing to substantially accommodate a curved surface. For example, in one embodiment, the device 100 may be capable of accommodating an uneven surface of a tissue (e.g., a mucosa). By way of further example, in one embodiment, the device 100 may be sufficiently flexible to be rolled and placed within a containment vehicle. By way of further example, in one embodiment, the device 100 may be rolled into a smaller configuration and placed into a containment vehicle suitable for oral administration. The device 100 may then be released in the subject (e.g., in the gastrointestinal tract) where the device 100 may unroll and adhere to a wall of the gastrointestinal tract (e.g., a mucosa). As discussed elsewhere herein, the device 100 may include one or more additives (e.g., plasticizers) that improve the flexibility of the device 100.

In some embodiments, the device 100 may adhere to a tissue via the opposing surface 112, and the adherence may be represented as a significant force. For example, adherence to the tissue may be measured via an adapted porcine tissue tensile assay. The adapted porcine tissue tensile assay provides a measurement for the amount of force required to pull the device off of a tissue. The measured force from the adapted porcine tissue tensile assay may be reported in millinewtons (mN). In one exemplary embodiment, the adapted porcine tissue tensile assay experiments may be performed to determine the adhesion force between the Opposing Surface and the intestinal site. The assay is conducted at 20 -25 degrees Celsius, 1 atm of pressure, and 50 - 60 percent relative humidity. As such, frozen pig intestine may be used in these studies. The intestine is rinsed with PBS and then cut into 5 cm long loops. The loop is then sliced open and laid flat in a shallow dish, lumen side facing up. 150 mM Phosphate-Buffered Saline is added to the dish to wet the tissue, but not submerge it. There should not be a layer of water greater than 1 mm on the surface of the tissue. The device 100 is placed onto the intestine sections, mucoadhesive-side down, with no pressure applied by the placement. The device 100 is allowed to incubate for 15 minutes. The dish containing the tissue is then placed on a balance. A small piece of a plastic cylinder (2 cm in length and 1 mm in diameter) is super-glued to the backing side of the device adhered to the tissue. The other end of the cylinder is attached to a string that passes over a pulley. The cylinder is gradually pulled until the device detaches from the mucosa. The detachment force (tensile force) at which the adhesive bond between the opposing surface 112 of the device 100 and the mucosa failed is recorded. For example, in one embodiment, the device 100 may adhere to a tissue with a tensile force of at least about 1 mN. By way of further example, in one embodiment, the device 100 may adhere to a tissue with a tensile force of at least about 2 mN. By way of further example, in one embodiment, the device 100 may adhere to a tissue with a tensile force of at least about 5 mN. By way of further example, in one embodiment, the device 100 may adhere to a tissue with a tensile force of at least about 10 mN. By way of further example, in one embodiment, the device 100 may adhere to a tissue with a tensile force of at least about 50 mN. By way of further example, in one embodiment, the device 100 may adhere to a tissue with a tensile force of at least about 100 mN. By way of further example, in one embodiment, the device 100 may adhere to a tissue with a tensile force of at least about 200 mN. By way of further example, in one embodiment, the device 100 may adhere to a tissue with a tensile force of at least about 500 nM.

In certain embodiments, adhesion of a device 100 to a tissue (e.g., an intestinal site, a mucosa, and the like) in the gastrointestinal system of a subject may be facilitated by peristalsis. Thus, in some embodiments, a device 100 may be administered with a peristalsis enhancer. Non-limiting examples of peristalsis enhancers include magnesium salts, senna, fiber, bisacodyl, and the like, and combinations thereof. In some cases, the peristalsis enhancer may be incorporated into the device. For example, in one embodiment, the peristalsis enhancer may be within the reservoir 102. In some instances, the peristalsis enhancer may be administered separately (i.e., prior to, concurrently with, or after administration of the device). In some embodiments, a peristaltic reflex of a subject may be leveraged. For example, in certain embodiments, the device 100 may be administered prior to, concurrently with, or after consumption of food by a subject. In some embodiments, the device 100 may be administered at least about 5 minutes, in some embodiments at least about 10 minutes, in some embodiments at least about 15 minutes, in some embodiments at least about 20 minutes, in some embodiments at least about 30 minutes, or in some embodiments at least about 60 minutes after consumption of food by a subject. In some embodiments, the device 100 may be administered between about 30 minutes and about 120 minutes, in some embodiments between about 30 minutes and about 60 minutes after consumption of food by a subject.

In some embodiments, the rate of agent release from the device 100 may be at least about 0.001 micrograms per hour, in some embodiments at least about 0.005 micrograms per hour, in some embodiments at least about 0.01 micrograms per hour, in some embodiments at least about 0.05 micrograms per hour, in some embodiments at least about 0.1 micrograms per hour, in some embodiments at least about 0.5 micrograms per hour, in some embodiments at least about 1 microgram per hour, in some embodiments at least about 5 micrograms per hour, in some embodiments at least about 10 micrograms per hour, in some embodiments at least about 20 micrograms per hour, in some embodiments at least about 50 micrograms per hour, in some embodiments at least about 100 micrograms per hour, in some embodiments at least about 500 micrograms per hour, in some embodiments at least about 1 mg per hour, and in some embodiments at least about 5 mg per hour. It should be understood that a device 100 may release an agent at any of these rates even if releasing the agent or drug for less than about 1 hour. In some embodiments, the rate of agent release may be essentially independent of the agent loading of the device 100. In some embodiments, the cumulative amount of agent released may increase directly as a function of the agent loading of the device.

In some cases, the device 100 may be capable of delivering an agent such that the concentration of the agent attains a level of between about 1 ng/mL and about 1 mg/mL, in some embodiments between about 10 ng/mL and about 1 mg/mL, in some embodiments between about 100 ng/mL and about 1 mg/mL, in some embodiments between about 1 microgram/mL and about 1 mg/mL, in some embodiments between about 10 micrograms/mL and about 1 mg/mL, in some embodiments between about 1 ng/mL and about 100 micrograms/mL, in some embodiments between about 1 ng/mL and about 10 micrograms/mL, and in some embodiments between about 1 ng/mL and about 1 microgram/mL.

Yet further still, in general, in some embodiments, a plurality of devices 100 may be provided. For example, in one embodiment, two devices 100 may be provided wherein a first device contains a first agent and a second device contains a second agent. In one embodiment, the first device is not the same as the second device. For example, in one embodiment, the first device may have a different configuration of elements compared to the second device. By way of further example, in one embodiment, the first device may include a backing element, a reservoir, and a mucoadhesive element, and the second device may include a backing element, a reservoir, a mucoadhesive element, and an enteric element. In another embodiment, the first agent may not be the same as the second agent. For example, in one embodiment, the first agent may be insulin, and the second agent may be calcitonin. By way of further example, in one embodiment, the plurality of devices may be substantially the same and the first and second agent may be different. By way of further example, in one embodiment, the plurality of devices may be substantially different and the first and second agent may be the same. In certain embodiments, a portion of a plurality of devices may be substantially the same (e.g., the backing element) and a portion of the plurality of devices may be substantially different (e.g., the enteric element). Further, described herein are systems for delivery of mucoadhesive devices, for example, for delivery of a plurality of devices. For example, in one embodiment, a system may include one or more mucoadhesive devices (e.g., wafers) configured for release of an agent. The system may, in some embodiments, include a plurality of devices encapsulated in a containment vehicle. For instance, the plurality of devices may be encapsulated in a capsule, a caplet, a gelcap, or a tablet. The containment vehicle may be configured to release one or more devices in a desired location, for example, the intestine. In some instances, the containment vehicle may include between 2 and 9 devices, in some embodiments between 11 and 15 devices, and in some embodiments, between 16 and 20 devices. In certain embodiments, the containment vehicle may contain 2, 3, 4, 5, 6, 7, 8, or 9 devices.

In some embodiments, an anti-adhesion layer or anti-adhesion agent may emit a gas. For example, in one embodiment, the anti-adhesion layer or anti-adhesion agent may effervesce upon contact with an aqueous environment. In some cases, the release of a gas may facilitate separation of the devices (e.g., wafers). An anti-adhesion layer or anti-adhesion agent that emits a gas may, in some embodiments, include a dispersal agent that includes a combination of a carbonate and an acid that may react to produce carbon dioxide gas upon contact with an aqueous solution. For example, in some cases, the carbonate may include bicarbonate. Non-limiting examples of carbonate counter ions or bicarbonate counter ions include sodium, potassium, magnesium, and calcium. In some embodiments, the acid may be any biocompatible acid capable of reacting with the carbonate to release a gas (e.g., carbon dioxide). In some cases, the acid may be a non-volatile acid. Non-limiting examples of acids include citric acid, ascorbic acid, lactic acid, and glycolic acid. The carbonate and acid may be present in any ratio suitable to produce effervescence. In some embodiments, the carbonate and acid may be present as a mixture to form the anti-adhesive agent, or as a mixture in the anti-adhesion layer. In other embodiments, the carbonate and acid may be in isolated in separate regions of the anti-adhesion layer.

Further, in general, a plurality of devices 100 may be placed and delivered within a dissolvable container which is under slight over-pressure. Upon dissolution of the container, the over-pressure pushes the devices away from each other, thereby minimizing self-aggregation.

In some embodiments, the device 100 may adhere to a tissue and form a seal. For example, in one embodiment, the device 100 may adhere to a tissue and form a seal that defines an inner privileged region of tissue that is at least partially isolated from a region of tissue outside of the inner privileged region of tissue. In some embodiments, the agent released -from the reservoir 102 through the mucoadhesive element 104 via the population of passageways 110 - within the privileged region may have substantially improved permeation of the agent compared to the agent being released to a tissue region outside the privileged region. For example, in one embodiment, delivery of the agent within the privileged region results in a higher concentration of the agent compared to delivery of the agent without the use of a privileged region. In some embodiments, substantially all of the privileged region of tissue is adhered to the device 100. In some embodiments, the mucoadhesive element of the device 100 may include a first region having reduced or substantially zero mucoadhesive properties, and a second region having mucoadhesive properties. In some embodiments, the second region may include an agent. In some embodiments, the first region may be substantially free of agent. In certain embodiments, the mucoadhesive element may have a ring configuration where a first region is surrounded by a second region. In some embodiments, the first region may be an ellipsoid. In other embodiments, the first region may be a cylinder. In certain embodiments, the first region may be round, oval, triangular, quadrangular, polygonal, or irregular. In some embodiments, a substance other than the agent may be released from the device 100 to further isolate the privileged region from the surrounding intestinal fluid. For example, in one embodiment, the substance may include an excipient in the form of an oil, a surfactant, a polymer, other hydrophobic media, and the like.

In certain embodiments, the seal formed by adhesion of the device 100 to a tissue may be capable of limiting or even substantially excluding infiltration of molecules from outside the privileged region to the privileged region. This property may be advantageous, for example, in the intestine where it may be desirable to prevent intraluminal materials from entering the privileged region and being absorbed by the subject. In some embodiments, molecules substantially unable to infiltrate the privileged region may have a molecular weight greater than about 50 Da, in some embodiments greater than about 100 Da, in some embodiments greater than about 200 Da, in some embodiments greater than about 500 Da, in some embodiments greater than about 1000 Da, in some embodiments greater than about 2000 Da, in some embodiments greater than about 5000 Da, and in some embodiments greater than about 10000 Da. In certain embodiments, an agent may be substantially prevented from escaping the privileged region to the intraluminal region.

In general, in certain embodiments, the adhesiveness of one device 100 for another device 100 may be reduced or substantially eliminated by the geometrical configuration of the device 100. For example, in some embodiments, the device 100 may have a non-planar shape, which assists in minimizing aggregation of the device. In some embodiments, the device 100 may be configured as a hemisphere, a cylinder, a rod, an ellipsoid, or a sphere, a doughnut, a toroid, a pyramid, a triangle, a star shape, an irregular shape, and the like.

In general, in some embodiments, at least a portion of the device 100 may swell as a function of pH, but for example, the mucoadhesive element 104 does not swell. For example, in one embodiment, the reservoir 102 containing the agent may swell when exposed to an aqueous environment having a pH of between about 1 and about 3, in some embodiments between about 3 and about 5, in some embodiments between about 5 and about 7, in some embodiments between about 7 and about 9, in some embodiments between about 1 and about 5, in some embodiments between about 3 and about 7, in some embodiments between about 5 and about 9, and in some embodiments between about 6 and about 8. In some instances, the rate of agent release is modulated by the swelling of the device 100. For example, in some instances, the agent release rate may increase upon swelling of the device. In other instances, the agent release may decrease upon swelling of the device 100.

Alternatively, the containment vehicle may also be enteric coated, such that the containment vehicle preferentially dissolves in the intestine, and the enteric coating layer on the device(s) further protects the device(s) until the layer is dissolved.

In some embodiments, an enteric cap may be used to cover the passageway openings of the passageways 110. For example, in one embodiment, the enteric caps or covers may be water soluble at pH 6.5. In other embodiments, two or more different enteric caps may be used to cover the passageways 110, For example, in one embodiment, a first enteric cap may dissolve preferentially in the stomach compared to a second enteric cap that may dissolve in the small intestine. By way of further example, in one embodiment, a first enteric cap may preferentially hydrolyze in the stomach compared to a second enteric cap that may hydrolyze in the small intestine. In other embodiments, the enteric caps may be covered by the enteric coating as described herein. For example, in one embodiment, an enteric cap may be solubilized or hydrolyzed at a pH of about 6.5 and the enteric coating may be solubilized or hydrolyzed at a pH of about 7. In other embodiments, a first enteric cap may be water soluble at a pH of about 6.5 and a second enteric cap may be water soluble at a pH of about 7. In some embodiments, the enteric cap may degrade at a pH as described above per unit time.

### Methods of Manufacturing

A device 100 may be manufactured by any suitable method. In certain embodiments, a device 100 may be manufactured under sterile conditions. In other embodiments, a device 100 may be sterilized prior to packaging the device. In certain embodiments, the device 100 may be sterilized prior to administration to a subject. In some embodiments, a device 100 may be manufactured using a process including salt leaching, solvent casting, molding, spray coating, spray drying, spin coating, and/or compression. Other methods will be known to those of ordinary skill in the art. In some embodiments, a coating may be applied to a device precursor and the coating compressed to form an element (e.g., a backing element 106 or a mucoadhesive element 104). In some embodiments, an element (e.g., a mucoadhesive element 104, a backing element 106, and/or a sacrificial element) may be applied using a spray-coating process. For example, in one embodiment, the backing element 106 may be fabricated by dissolving backing element components in a volatile solvent and applying the mixture to all but one side of the device via solvent evaporation. By way of further example, in one embodiment, the mucoadhesive element 104 may be fabricated by dissolving mucoadhesive element components in a volatile solvent and applying the mixture last via solvent evaporation. In some embodiments, a layer of the mucoadhesive element or material may be coated on a device 100 by dissolving a layer of material in an appropriate solvent (e.g., water) and applying the resultant solution onto the device. The coating may be applied using any suitable technique, such as spraying. Alternatively, an element or layer may be applied in dry form. For example, solid powder of an element or layer material may be applied to a device and compressed to form a layer. By way of further example, in one embodiment, the reservoir element may be fabricated by mixing reservoir components with agent components and compressing both said components using, for example, a hydraulic press. In one embodiment, this "pre-fabricated" reservoir element may then be subsequently incorporated with the other elements of the device 100 as would be appreciated by those of skill in the art. By way of further example, in one embodiment, the push element 114 may be fabricated by mixing push element components and compressing said push element components, for example, with a hydraulic press. In one embodiment, this "pre-fabricated" push element 114 may then be subsequently incorporated with other elements of the device 100 as would be appreciated by those of skill in the art. In some embodiments, the reservoir 102 may be prepared first and the backing element 106 and any additional element applied to the reservoir 102. In other embodiments, two or more elements or components of a device 100 may be prepared and then assembled. For instance, a backing element shell may be prepared and a reservoir 102 may be placed into the backing element shell to form a device 100. The backing element shell and reservoir 102 may, in some cases, be bonded using any suitable method. For example, in some embodiments a backing element and a reservoir 102 may be bonded using an adhesive or compression.

In some cases, a device 100 may be manufactured on a surface. For example, a reservoir 102 may be deposited on a surface and one or more coatings may be applied to the reservoir 102 to create a device 100. In some instances, the device 100 may be removed from the surface such that the portion of the device 100 that was in contact with the surface is essentially free of the coating. In one non-limiting example, a device 100 may have a first side and a second side (e.g., a front side and a back side). The device 100 may be positioned on a surface such that the first side is in contact with the surface. One or more coatings may then be applied to the device onto the second side. The first side may be shielded from the coating since the first side is in contact with the surface. In some instances, a coating may coat all sides of the device 100 not in contact with the surface. The device 100 may then be removed from the surface. Such a method may be used, for example, to construct a device 100 by successively applying elements or layers to a surface. In some cases, material for manufacturing the device 100 may be deposited in discrete areas of a surface, where a device 100 is manufactured at each discrete area. In other embodiments, surfaces larger than an individual device 100 may be coated successively, and individual devices may be cut out of the resultant layered construct (e.g., with a hole-punch).

In some embodiments, the holes at the end of the passageways 110, as described herein, may be fabricated using mechanical methods. For example, in one embodiment, mechanical methods may include drilling, punching, scraping, piercing, and the like. By way of further example, in one embodiment, drilling may include handle-drilling, lazer drilling, and the like. By way of further example, in one embodiment, scraping may include using a needle, a razor blade, and the like. By way of further example, in one embodiment, piercing may include using a needle, and the like.

### Pharmaceutical Compositions

In another aspect, the devices 100 as contemplated herein may be formulated together with a pharmaceutically acceptable carrier. In particular, the present disclosure provides devices 100 formulated together with one or more pharmaceutically acceptable carriers. The most suitable form of administration in any given case will depend on the degree and severity of the condition being treated and on the nature of the particular agent being used. For example, the devices may be formulated as a unit dose and/or may be formulated for oral administration.

Exemplary devices 100 may be used in the form of a pharmaceutical preparation, for example, in solid, semisolid, or liquid form, which contains the devices, in admixture with an organic or inorganic carrier or excipient suitable for external, enteral, or parenteral applications. The devices 100 may be combined, for example, with the usual non-toxic, pharmaceutically acceptable carriers for tablets, pellets, capsules, suppositories, solutions, emulsions, suspensions, and any other form suitable for use. As the devices 100 can vary in size from, for example, nanoscale dimensions to millimeters or even larger, the size of the devices may be considered when contemplating a formulation of the devices. For example, the devices 100 may be prepared as a suspension in a liquid formulation. In other instances, the devices 100 may be trapped in a gel formulation. In still other examples, the devices 100 may be contained in a solid formulation, such as a capsule. In some embodiments, the agent may be included in the devices 100 in an amount sufficient to produce the desired effect upon the process or condition of the disease.

For preparing solid formulations of devices 100 such as tablets, the devices 100 may be mixed with a pharmaceutical carrier, for example, conventional tableting ingredients such as corn starch, lactose, sucrose, sorbitol, talc, stearic acid, magnesium stearate, dicalcium phosphate or gums, and other pharmaceutical diluents, for example, water, to form a solid pre-formulation composition containing a heterogeneous mixture of devices 100 and one or more carriers. When referring to these pre-formulation compositions as homogeneous, it is meant that the devices 100 are dispersed evenly throughout the composition so that the composition may be readily subdivided into equally effective unit dosage forms such as tablets, pills and capsules.

In solid dosage forms for oral administration (e.g., capsules, tablets, pills, dragees, powders, granules, and the like), the devices 100 may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, for example, acetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the compositions may also include buffering agents. Solid compositions of a similar type may also be employed as fillers in soft and hard-filled gelatin capsules using such excipients as lactose or milk sugars, as well as high molecular weight polyethylene glycols, and the like.

A tablet may be made by compression or molding, optionally with one or more accessory ingredients. Compressed tablets may be prepared using binder (e.g., gelatin or hydroxypropylmethyl cellulose), lubricant, inert diluent, preservative, disintegrant (e.g., sodium starch glycolate or cross-linked sodium carboxymethyl cellulose), surface-active or dispersing agent. Molded tablets may be made by molding in a suitable machine a mixture of the devices moistened with an inert liquid diluent. Tablets, and other solid dosage forms, such as dragees, capsules, pills, and granules, may optionally be scored or prepared with coatings and shells, such as enteric coatings and other coatings well known in the pharmaceutical-formulating art.

The devices 100 may, in some embodiments, be formulated for inhalation or insufflation and include solutions and suspensions in pharmaceutically acceptable, aqueous or organic solvents, or mixtures thereof, and powders, provided that they have dimensions suitable for inhalation or insufflation. Such formulations may be advantageous, for example, for delivery of devices to a mucosa in the nasal passage or sinuses.

Liquid dosage forms for oral administration of the devices include pharmaceutically acceptable emulsions, micro emulsions, solutions, suspensions, syrups, and elixirs. In addition to the devices, the liquid dosage forms may contain inert diluents commonly used in the art, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, cyclodextrins, and mixtures thereof. In certain embodiments, the devices 100 may include a coating (e.g., an enteric coating) that substantially inhibits release of the agent while the devices are in a liquid dosage formulation. In other embodiments, the liquid dosage formulations may be formulated to substantially prevent release of the agent from the devices. For example, the liquid formulation may have a pH range that differs from the pH range in which agent is released from the devices.

Suspension of devices 100 may be facilitated by addition of suspending agents, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing devices with one or more suitable nonirritating excipients or carriers including, for example, cocoa butter, polyethylene glycol, a suppository wax or a salicylate, and which is solid at room temperature, but liquid at body temperature and, therefore, will melt in the body cavity and release the devices.

Ointments, pastes, creams and gels may contain, in addition to the devices, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to the devices, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane.

Pharmaceutical compositions suitable for parenteral administration include devices in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Parenteral administration of the devices may allow the devices to adhere to the gut mucosa and deliver an agent.

Examples of suitable aqueous and non-aqueous carriers which may be employed in the pharmaceutical compositions include water, ethanol, polyols (e.g., glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required device size in the case of dispersions, and by the use of surfactants.

In another aspect, enteral pharmaceutical formulations including the devices, an enteric material, and a pharmaceutically acceptable carrier or excipient thereof are provided. Enteric materials refer to polymers that are substantially insoluble in the acidic environment of the stomach, and that are predominantly soluble in intestinal fluids at specific pHs. The small intestine is the part of the gastrointestinal tract (i.e., the gut) between the stomach and the large intestine, and includes the duodenum, jejunum, and ileum. The pH of the duodenum is about 5.5, the pH of the jejunum is about 6.5, and the pH of the distal ileum is about 7.5. Accordingly, enteric materials are not soluble, for example, until a pH of about 5.0, of about 5.2, of about 5.5, of about 5.6, of about 5.8, of about 6.0, of about 6.2, of about 6.4, of about 6.5, of about 6.8, of about 7.0, of about 7.2, of about 7.4, of about 7.5, of about 7.8, of about 8.0, of about 8.2, of about 8.4, of about 8.6, of about 8.8, of about 9.0, of about 9.2, of about 9.4, of about 9.6, of about 9.8, or of about 10.0. Exemplary enteric materials include cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate butyrate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (e.g., Eudragit L30D55, Eudragit FS30D, Eudragit L100, Eudragit S100, Kollicoat EMM30D, Estacryl 30D, Coateric, and Aquateric). The solubility of each of the above materials is either known or is readily determinable in vitro. For example, the foregoing is a list of possible materials, but one of skill in the art with the benefit of the instant disclosure would recognize that the foregoing list is not comprehensive and that there are other enteric materials that may be used.

Advantageously, kits are provided for use by, for example, a diabetic in need of blood glucose concentration control. Such kits include a suitable dosage form of devices such as those described above and instructions describing the method of using such devices to control blood glucose concentration. The instructions would direct the consumer or medical personnel to administer the dosage form according to administration modes known to those skilled in the art. Such kits could advantageously be packaged and sold in single or multiple kit units. An example of such a kit is a so-called blister pack. Blister packs are well known in the packaging industry and are widely used for the packaging of pharmaceutical unit dosage forms (e.g., tablets, capsules, and the like, comprising devices).

### Method of Treatment

In some embodiments, a device 100 may be administered to an individual, patient, or a subject. In some cases, the device 100 may be administered as a single device. In other embodiments, a plurality of devices 100 may be administered. As described herein, in some embodiments, a device 100 may be administered in a containment vehicle. It should be understood that the device 100 may be an isolated device or may be a member of a plurality of devices.

Alternatively, the devices and systems described herein may be administered to a subject in need thereof without food or under a fasting condition. For example, the device may be administered at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, between about 3 hours to about 12 hours, between about 4 hours to about 12 hours, between about 4 hours to about 10 hours, between about 4 hours to about 8 hours, or between about 4 hours to about 6 hours, after consumption of food by a subject.

Alternatively, the devices and systems described herein may be administered to a subject in need thereof under a condition of fluid restriction. This restriction shall mean that over the stated time, the subject may consume less than 16 oz. of fluids, less than 8 oz of fluids, less than 4 oz of fluids, less than 2 oz of fluids, or less than 1 oz of fluids. For example, the subject may be restricted in their consumption of fluids prior to being administered the device for at least about 1 hours, at least about 2 hours, at least about 3 hours, at least about 4 hours, at least about 5 hours, at least about 8 hours, between about 1 hours to about 2 hours, between about 1 hours to about 4 hours. Additionally, the subject may be restricted in their consumption of fluids after being administered the device for at least about 1 hours, at least about 2 hours, at least about 3 hours, at least about 4 hours, at least about 5 hours, at least about 8 hours, between about 1 hours to about 2 hours, between about 1 hours to about 4 hours.

Treatment can be continued for as long or as short of a period as desired. The devices or systems may be administered on a regimen of, for example, one to four or more times per day. A suitable treatment period can be, for example, at least about one week, at least about two weeks, at least about one month, at least about six months, at least about 1 year, or indefinitely. A treatment period can terminate when a desired result is achieved. A treatment regimen can include a corrective phase, during which a dose sufficient, for example, to reduce symptoms is administered, and can be followed by a maintenance phase, during which a lower dose sufficient to maintain the reduced symptoms is administered. A suitable maintenance dose is likely to be found in the lower parts of the dose ranges provided herein, but corrective and maintenance doses can readily be established for individual subjects by those of skill in the art without undue experimentation, based on the disclosure herein.

In certain embodiments, the device or system may be used to deliver an agent (e.g., insulin) to a subject in need thereof. In some embodiments, the device or system may be capable of delivering insulin such that the blood glucose concentration of a subject may be maintained within a range of 3 mmol/L to 8 mmol/L for at least about 1 hour, in some embodiments at least about 2 hours, in some embodiments at least about 4 hours, in some embodiments at least about 8 hours, in some embodiments at least about 10 hours, in some embodiments at least about 12 hours, in some embodiments at least about 16 hours, in some embodiments at least about 20 hours, in some embodiments at least about 24 hours, in some embodiments at least about 30 hours, in some embodiments at least about 36 hours, and in some embodiments at least about 48 hours.

In certain embodiments, the device or system may be used to deliver an agent (e.g., calcitonin) to a subject in need thereof. For example, the device or system may be used to treat hypercalcemia. In another example, the device or system may be used to treat a bone disease, such as osteoporosis. In yet another embodiment, the device or system may be used to treat a mental disorder, such as bipolar disorder or mania. In some embodiments, the device or system may be capable of delivering calcitonin such that the plasma calcium concentration of a subject may be reduced after a period of about 1 hour by about 5% to about 50%, in some embodiments about 5% to about 25%, in some embodiments about 10% to about 25%, in some embodiments about 5% to about 10%, in some embodiments about 10% to about 50%, in some embodiments about 15% to about 50%, in some embodiments about 25% to about 50%, and in some embodiments about 30% to about 50%, as compared to the plasma calcium concentration of the subject as measured prior to treatment. In certain embodiments, the reduction in plasma calcium concentration may persist for a period of at least about 1 hour, in some embodiments at least about 2 hours, in some embodiments at least about 4 hours, in some embodiments at least about 8 hours, in some embodiments at least about 10 hours, in some embodiments at least about 12 hours, in some embodiments at least about 16 hours, in some embodiments at least about 20 hours, in some embodiments at least about 24 hours, in some embodiments at least about 30 hours, in some embodiments at least about 36 hours, and in some embodiments at least about 48 hours.

The devices and systems described herein may be used to administer an agent to patients (e.g., animals and/or humans) in need of such treatment in dosages that will provide optimal pharmaceutical efficacy. It will be appreciated that the number and/or type of devices or systems required for use in any particular application will vary from patient to patient, not only with the particular agent selected, but also with the concentration of agent in the devices, the route of administration (e.g., oral, nasal, vaginal, rectal, and the like), the nature of the condition being treated, the age and condition of the patient, concurrent medication or special diets then being followed by the patient, and other factors which those skilled in the art will recognize, with the appropriate dosage ultimately being at the discretion of the attendant physician. For example, in one embodiment, the dosage may include multiple agents within the reservoir of the device and may include multiple (i.e., identical or non-identical) release rates for one or more of the agents. For treating clinical conditions and diseases, a compound (i.e., an agent) may be administered, for example, orally, parenterally, by inhalation spray, or rectally in dosage unit formulations containing conventional nontoxic pharmaceutically acceptable carriers, adjuvants, and vehicles. Parenteral administration may include subcutaneous injections, intravenous or intramuscular injections, or infusion techniques. In some embodiments, the dosage form may include the device itself. In some embodiments, the treatment of a patient may include an intestinal pretreatment. For example, in one embodiment, the intestinal pretreatment may include administration of an agent or agents to improve the adhesive characteristics of the intestine. Exemplary agents that improve the adhesive characteristics of the intestine may include mucolytics, expectorants, drying agents, swellable polymers, biologics, prokinetics, sorbents, and the like, and combinations thereof.

### EXAMPLES

### EXAMPLE 1

| **Component** | **Amount (%)** |
|---|---|
| **Reservoir** | |
| Sucrose | 72 |
| Pectin | 8 |
| Octreotide | 20 |

| **Push** | |
|---|---|
| Polyethylene Oxide (M_{w} 8,000,000) | 60 |
| Sodium chloride | 40 |

| **Mucoadhesive** | |
|---|---|
| Carbopol 974 | 25 |
| Pectin | 25 |
| Sodium Carboxymethylcellulose | 50 |

| **Backing** | |
|---|---|
| Polyethylene oxide (Mn = 400) | 1.25 |
| Cellulose Acetate | 3.75 |
| Acetone | 95 |

110 mg of reservoir is weighed out and placed in a 13 mm diameter flat press insert. Reservoir is pressed at 3500 LBF for 5 minutes. Using biopsy punches, 4 mm disks are punched out of the resultant 13 mm disk. 50 mg of mucoadhesive element is then added to the 13 mm diameter press insert. Three of the resultant disks are placed on the mucoadhesive element in an evenly spaced manner. 150 mg of the push element is carefully poured on top of the reservoir disks. The material is then pressed at 3500 LBF for 5 minutes. 7 mm disks are then cut out of resultant 13 mm disks. Devices are then dip-coated in backing element and air-dried. Once dry, a 3 mm orifice is drilled through the mucoadesive element and backing element into the reservoir using a standard drill bit.

### EXAMPLE 2

| **Component** | **Amount (%)** |
|---|---|
| **Reservoir** | |
| Sucrose | 72 |
| Pectin | 8 |
| Octreotide | 20 |

| **Push** | |
|---|---|
| Polyethylene Oxide (M_{w} 8,000,000) | 60 |
| Sodium chloride | 40 |

| **Mucoadhesive** | |
|---|---|
| Carbopol 974 | 25 |
| Pectin | 25 |
| Sodium Carboxymethylcellulose | 50 |

| **Backing** | |
|---|---|
| Polyethylene oxide (Mn = 400) | 1.25 |
| Cellulose Acetate | 3.75 |
| Acetone | 95 |

75 mg of mucoadhesive element is weighed out and placed in a 13 mm diameter flat press insert. Mucoadhesive element is pressed at 3500 LBF for 5 minutes. 75 mg of reservoir is then added to the 13 mm diameter press insert. The bilayer composition is pressed at 3500 LBF for 5 minutes. 75 mg of push element is carefully poured above the bilayer reservoir disks. The material is then pressed at 3500 LBF for 5 minutes. 7 mm disks are then cut out of the resultant 13 mm disk. Devices are then dip-coated in backing element and air-dried. Once dry, a 3 mm orifice is drilled through the mucoadesive element and backing element into the reservoir using a standard drill bit.

### EXAMPLE 3

| **Component** | **Amount (%)** |
|---|---|
| **Reservoir** | |
| Sucrose | 72 |
| Pectin | 8 |
| Octreotide | 20 |

| **Mucoadhesive** | |
|---|---|
| Carbopol 974 | 25 |
| Pectin | 25 |
| Sodium Carboxymethylcellulose | 50 |

| **Backing** | |
|---|---|
| Polyethylene oxide (Mn = | 1.25 |
| 400) | |
| Cellulose Acetate | 3.75 |
| Acetone | 95 |

75 mg of mucoadhesive element is weighed out and placed in a 13 mm diameter flat press insert. Mucoadhesive element is pressed at 3500 LBF for 5 minutes. 75 mg of reservoir is then added to the 13 mm diameter press insert. The bilayer composition is pressed at 3500 LBF for 5 minutes. 7 mm disks are then cut out of the resultant 13 mm disk. Devices are then dip-coated in backing element and air-dried. Once dry, a 3 mm orifice is drilled through the mucoadesive element and backing element into the reservoir using a standard drill bit.

### EXAMPLE 4

| **Component** | **Amount (%)** |
|---|---|
| **Reservoir** | |
| Sucrose | 72 |
| Pectin | 8 |
| Octreotide | 20 |

| **Mucoadhesive** | |
|---|---|
| Carbopol 974 | 25 |
| Pectin | 25 |
| Sodium Carboxymethylcellulose | 50 |

| **Backing** | |
|---|---|
| Polyethylene oxide (Mn = 400) | 1.25 |
| Cellulose Acetate | 3.75 |
| Acetone | 95 |

75 mg of mucoadhesive element is weighed out and placed in a 13mm diameter flat press insert. Mucoadhesive element is pressed at 3500 LBF for 5 minutes. 75 mg of reservoir is then added to the 13 mm diameter press insert. The bilayer composition is pressed at 3500 LBF for 5 minutes. 7 mm disks were then cut out of resultant 13mm disk. Devices are then dip-coated in backing element and air-dried. Once dry, a 1.5 mm orifice is drilled through the mucoadhesive element and backing element into the reservoir using a standard drill bit.

### EXAMPLE 5

| **Component** | **Amount (%)** |
|---|---|
| **Reservoir** | |
| Sucrose | 72 |
| Pectin | 8 |
| Octreotide | 20 |
| **Push** | |
| Polyethylene Oxide (M_{w} 8,000,000) | 60 |
| Sodium chloride | 40 |

| **Mucoadhesive** | |
|---|---|
| Carbopol 974 | 4 |
| Polyethylene oxide (Mn = 400) | 96 |

| **Backing** | |
|---|---|
| Polyethylene oxide (Mn = 400) | 1.25 |
| Cellulose Acetate | 3.75 |
| Acetone | 95 |

100 mg of reservoir element is weighed out and placed in a 13mm diameter flat press insert. Reservoir element is pressed at 1000 LBF for 5 minutes. 100 mg of push element is then added to the 13 mm diameter press insert. The bilayer composition is pressed at 5000 LBF for 5 minutes. 5 mm disks are then cut out of resultant 13mm disk. Devices are then dip-coated in backing element and air-dried. Once dry, a 1.5 mm orifice is drilled through the backing element into the reservoir using a standard drill bit. A coat of the mucoadhesive element is then applied to the devices using a paint brush.

### EXAMPLE 6

| **Component** | **Amount (%)** |
|---|---|
| **Reservoir** | |
| Sucrose | 72 |
| Pectin | 8 |
| Octreotide | 20 |
| **Push** | |
| Polyethylene Oxide (M_{w} 8,000,000) | 60 |
| Sodium chloride | 40 |

| **Mucoadhesive** | |
|---|---|
| Carbopol 974 | 25 |
| Pectin | 25 |
| Sodium Carboxymethylcellulose | 50 |

| **Backing** | |
|---|---|
| Polyethylene oxide (Mn = 400) | 1.25 |
| Cellulose Acetate | 3.75 |
| Acetone | 95 |

110mg of reservoir element is weighed out and placed in a 13mm diameter flat press insert. Reservoir element is pressed at 3500 LBF for 5 minutes. Using biopsy punches, 4mm disks are punched out of resultant 13mm disk. 50 mg of mucoadhesive element is then added to the 13 mm diameter press insert. Three of the resultant disks are placed on a layer of the mucoadhesive element in an evenly spaced manner. 150mg of push element is carefully poured on top of the disks. The material is then pressed at 3500 LBF for 5 minutes. 7mm disks are then cut out of resultant 13mm disk. Devices are then dip-coated in backing element and air-dried. Once dry, a 3mm orifice is drilled through the backing element and mucoadhesive element into the reservoir using a standard drill bit.

### EXAMPLE 7

| **Component** | **Amount (%)** |
|---|---|
| **Reservoir** | |
| Sucrose | 72 |
| Pectin | 8 |
| Octreotide | 20 |

| **Mucoadhesive** | |
|---|---|
| Carbopol 974 | 4 |
| Polyethylene oxide (Mn = 400) | 96 |

| **Backing** | |
|---|---|
| Polyethylene oxide (Mn = 400) | 1.25 |
| Cellulose Acetate | 3.75 |
| Acetone | 95 |

110mg of reservoir element is weighed out and placed in a 13mm diameter flat press insert. Reservoir element is pressed at 5000 LBF for 5 minutes. Using biopsy punches, 5mm disks are punched out of the resultant 13mm disk. Devices are then dip-coated in backing element. Once dry, a 1.5mm orifice is drilled through the backing element using a standard drill bit. A coat of the mucoadhesive element is then applied to the disks using a paint brush.

### EXAMPLE 8

The present example demonstrates % bioavailabilities for devices according to the present disclosure, as well as comparative dosage forms.

To determine bioavailability, mucoadhesive devices according to aspects of the present disclosure, as well as comparative dosage forms, are tested by a surgical model (Surgical Bioavailability Assay).

Surgical Bioavailability Assay. In the Surgical Bioavailability Assay, a porcine model is used, in which the animals undergo open surgery such that devices and/or dosage forms to be tested can be placed at regions of the intestine of interest, such as the jejunum and ileum. After administration of anesthesia, a ventral midline incision is made to gain access to the animal's abdominal cavity. The jejunum and ileum are exposed, and incisions are made to manually place the devices and/or dosage forms being tested. After placement of the device and/or dosage form is complete, the intestinal incisions are closed, and the midline incision can also be temporarily closed. Also, access can be made for blood collection via the carotid artery or jugular vein. The animal can be kept under anesthesia for up to 4 hours to allow for blood collection, such as at 5 mins, 15 mins, 30 mins, 60 mins, 90 mins, and 2 hours after oral dosage form placement. Blood samples can also be collected prior to the surgical procedure. The percent bioavailability of the active agent provided with the devices and/or dosage forms is determined on the basis of the active agent levels detected in these blood samples.

FIG. 23 shows results for a Surgical Bioavailability Assay for the administration of octreotide in a mucoadhesive device according to the present disclosure prepared according to Example 7 above, compared to a solution of octreotide administered via intestinal instillation, and control wafers prepared as described in Gupta et al. (Mucoadhesive Intestinal Devices for Oral Delivery of Salmon Calcitonin, Journal of Controlled Release, 172 (2013) 753-762), As described in Gupta, the control wafers were prepared by compressing a polymeric matrix containing carbopol, pectin and sodium carboxymethylcellulose (1:1:2), and coating with an impermeable ethyl cellulose backing layer on all but one side.

As shown in FIG. 23, the mucoadhesives device prepared according to Example 7 of the instant disclosure exhibited significantly higher bioavailability than either the octreotide solution alone, or octreotide administered via the control wafer prepared according to Gupta. In particular, the mucoadhesive devices prepared according to Example 7 of the instant disclosure exhibited a mean bioavailability of over about 8%, whereas the octreotide solution and control wafers prepared according to Gupta exhibited a mean bioavailability close to 1%. Accordingly, the mucoadhesive devices according to aspects of the present disclosure may be capable of significantly enhancing the bioavailability of agents, including agents comprising peptides such as octreotide.

In further embodiments, numbered 1-57 below, aspects of the present disclosure include:
Embodiment 1. A device for the delivery of an agent to an intestinal site, the device comprising a backing element, a mucoadhesive element for adhering the device to the intestinal site, and a reservoir comprising the agent, wherein the mucoadhesive element comprises a polymer, an opposing surface having the capacity to adhere to the intestinal site, and a population of passageway(s) extending from the reservoir to the opposing surface for delivery of the agent from the reservoir to the intestinal site, each of the passageway(s) having a minimum diameter greater than 10 microns, the diameter being determined by cryogenic scanning electron microscopy after 30 minutes of hydration at 20 °C in phosphate buffered saline at pH 6.5.
Embodiment 2. A device for the delivery of an agent to an intestinal site, the device comprising a backing element, a mucoadhesive element for adhering the device to the intestinal site, a push element for induction of convective flow of the agent to the intestinal site, and a reservoir comprising the agent, wherein the agent comprises molecules having a molecular weight of at least 100 Da, the push element comprises an osmagent, the mucoadhesive element comprises a polymer, an opposing surface having the capacity to adhere to the intestinal site, and a population of passageway(s) extending from the reservoir to the opposing surface for delivery of the agent from the reservoir to the intestinal site that are permeable to said molecules.
Embodiment 3. A device for the delivery of an agent to an intestinal site, the device comprising a backing element, a mucoadhesive element for adhering the device to the intestinal site, and a reservoir comprising the agent, wherein the agent comprises a population of particles having a weight average particle size, P_{avg}, the mucoadhesive element comprises a polymer, an opposing surface having the capacity to adhere to the intestinal site, and a population of passageway(s) extending from the reservoir to the opposing surface for the delivery of the agent from the reservoir to the intestinal site wherein the population has a number average minimum diameter, D_{avg}, of at least 10 microns, and the ratio of D_{avg} to P_{avg} is at least 2.
Embodiment 4. A device for the delivery of an agent to an intestinal site, the device comprising a backing element, a mucoadhesive element for adhering the device to the intestinal site, and a reservoir comprising the agent, wherein the agent comprises a population of particles having a weight average particle size, P_{avg}, of at least 50 nm, the mucoadhesive element comprises a polymer, an opposing surface having the capacity to adhere to the intestinal site, and a population of passageway(s) extending from the reservoir to the opposing surface for the delivery of the agent from the reservoir to the intestinal site, wherein the population has at least one member having a minimum diameter of at least 10 microns.
Embodiment 5. The device of any preceding Embodiment wherein the release rate of the agent is at least 20% within 2 hours in a USP Dissolution Assay 711 with Apparatus 1 and a dissolution medium of 150 mM phosphate buffered saline at pH 6.5.
Embodiment 6. The device of any preceding claim Embodiment wherein the release rate of the agent is at least 50% within 2 hours in a USP Dissolution Assay 711 with Apparatus 1 and a dissolution medium of 150 mM phosphate buffered saline at pH 6.5.
Embodiment 7. The device of any preceding Embodiment wherein the release rate of the agent is at least 80% within 2 hours in a USP Dissolution Assay 711 with Apparatus 1 and a dissolution medium of 150 mM phosphate buffered saline at pH 6.5.
Embodiment 8. The device of any preceding Embodiment wherein the release rate of the agent is at least 90% within 2 hours in a USP Dissolution Assay 711 with Apparatus 1 and a dissolution medium of 150 mM phosphate buffered saline at pH 6.5.
Embodiment 9. The device of any preceding Embodiment wherein the release rate of the agent is at least 95% within 2 hours in a USP Dissolution Assay 711 with Apparatus 1 and a dissolution medium of 150 mM phosphate buffered saline at pH 6.5.
Embodiment 10. The device of any preceding Embodiment wherein the release rate of the agent is at least 99% within 2 hours in a USP Dissolution Assay 711 with Apparatus 1 and a dissolution medium of 150 mM phosphate buffered saline at pH 6.5.
Embodiment 11. The device of any preceding Embodiment wherein the release rate of the agent is at least 99.9% within 2 hours in a USP Dissolution Assay 711 with Apparatus 1 and a dissolution medium of 150 mM phosphate buffered saline at pH 6.5.
Embodiment 12. The device as in any preceding Embodiment wherein the opposing surface adheres to the intestinal site with a tensile adhesive strength of at least about 10 mN when measured using an adapted porcine tissue tensile assay.
Embodiment 13. The device as in any preceding Embodiment wherein the mucoadhesive element has an average dry thickness of less than about 100 µm to less than about 800 µm, measured in a direction perpendicular to the opposing surface.
Embodiment 14. The device of any preceding Embodiment wherein a fraction of the passageway population members provide a substantially linear pathway from the reservoir to the opposing surface.
Embodiment 15. The device of any preceding Embodiment wherein a fraction of the passageway population members provide a tortuous pathway from the reservoir to the opposing surface.
Embodiment 16. The device of any preceding Embodiment wherein the passageway population is a population having only one member.
Embodiment 17. The device of any of Embodiment s 1 to 10 wherein the passageway population comprises between 1 and 100 members.
Embodiment 18. The device of any of Embodiments 1 to 10 wherein the passageway population comprises at least 100 members.
Embodiment 19. The device of any preceding Embodiment wherein the population members are covered by an enteric material that inhibits release of the agent from the device before the device reaches the intestinal site.
Embodiment 20. The device of any preceding Embodiment wherein the population members are covered by an enteric material that inhibits release of the agent from the device before the device reaches the intestinal site and the enteric material is substantially insoluble at a pH less than 5.5.
Embodiment 21. The device of any preceding Embodiment wherein the population members are covered by an enteric material that inhibits release of the agent from the device before the device reaches the intestinal site and the enteric material is substantially insoluble at a pH less than 6.0.
Embodiment 22. The device of any preceding Embodiment wherein the population members are covered by an enteric material that inhibits release of the agent from the device before the device reaches the intestinal site and the enteric material is substantially insoluble at a pH less than 6.5.
Embodiment 23. The device of any preceding Embodiment wherein the population members are covered by an enteric material that inhibits release of the agent from the device before the device reaches the intestinal site and the enteric material is substantially insoluble at a pH less than 7.0.
Embodiment 24. The device of any preceding Embodiment wherein the population members are covered by an enteric material that inhibits release of the agent from the device before the device reaches the intestinal site and the enteric material is substantially insoluble at a pH less than 7.5.
Embodiment 25. The device of any preceding Embodiment wherein the device has an outer surface having a total surface area of about 1 to about 10 cm².
Embodiment 26. The device of any preceding Embodiment wherein the opposing surface has a surface area of about 0.5 to about 1 cm².
Embodiment 27. The device of any preceding Embodiment wherein the device has an outer surface having a total surface area and the opposing surface comprises about 30% of the total surface area of the device.
Embodiment 28. The device of any preceding claim Embodiment wherein the device has an outer surface having a total surface area and the opposing surface comprises about 40% of the total surface area of the device.
Embodiment 29. The device of any preceding Embodiment wherein the device has an outer surface having a total surface area and the opposing surface comprises about 50% of the total surface area of the device.
Embodiment 30. The device of any preceding Embodiment wherein the device has an outer surface having a total surface area and the opposing surface comprises about 60% of the total surface area of the device.
Embodiment 31. The device of any preceding Embodiment wherein the device has an outer surface having a total surface area and the opposing surface comprises about 70% of the total surface area of the device.
Embodiment 32. The device of any preceding Embodiment wherein the device has an outer surface having a total surface area and the opposing surface comprises about 80% of the total surface area of the device.
Embodiment 33. The device of any preceding Embodiment wherein the device has an outer surface having a total surface area and the opposing surface comprises about 90% of the total surface area of the device.
Embodiment 34. The device of any preceding Embodiment wherein the device has an outer surface having a total surface area and the opposing surface comprises about 95% of the total surface area of the device.
Embodiment 35. The device of any preceding Embodiment wherein the device has an outer surface having a total surface area and the opposing surface comprises about 99% of the total surface area of the device.
Embodiment 36. The device of any preceding Embodiment wherein the device has a thickness in the range of 100 microns to 5 mm.
Embodiment 37. The device of any preceding Embodiment wherein the reservoir further comprises an excipient.
Embodiment 38. The device of any preceding Embodiment wherein the reservoir further comprises an excipient and the excipient is selected from the group consisting of stabilizers, glidants, bulking agents, anti-adherents, disintegrants, binders, sorbents, preservatives, cryoprotectants, permeation enhancers, hydrating agents, enzyme inhibitors, and mucus modifying agents.
Embodiment 39. The device of any preceding Embodiment wherein the device comprises an osmagent and the osmagent is selected from the group consisting of water-soluble salts, carbohydrates, small molecules, amino acids, and hydrogel forming polymers.
Embodiment 40. The device of any preceding Embodiment wherein the mucoadhesive element has a swelling ratio of at most 1.05 as measured by cryo-SEM and SEM.
Embodiment 41. The device of any preceding Embodiment wherein the mucoadhesive element has a swelling ratio of at most 1.1 as measured by cryo-SEM and SEM.
Embodiment 42. The device of any preceding Embodiment wherein the mucoadhesive element has a swelling ratio of at most 1.2 as measured by cryo-SEM and SEM.
Embodiment 43. The device of any preceding Embodiment wherein the mucoadhesive element has a swelling ratio of at most 1.5 as measured by cryo-SEM and SEM.
Embodiment 44. The device of any preceding Embodiment wherein the mucoadhesive element has a swelling ratio of at most 2 as measured by cryo-SEM and SEM.
Embodiment 45. The device of any preceding Embodiment wherein the device comprises a push element, the push element has a swelling ratio of at least 2 to at least 4 as measured by cryo-SEM and SEM.
Embodiment 46. The device of any preceding Embodiment wherein the push element and the mucoadhesive element are on opposing sides of the reservoir and an osmotic pressure is generated to induce convective flow of the agent from the reservoir to the opposing surface via the population of passageway(s).
Embodiment 47. The device of any preceding Embodiment wherein the push element comprises a plurality of compartments configured for controlled induction of convective flow of the agent through the population of passageway(s) to the opposing surface.
Embodiment 48. The device of any preceding Embodiment wherein the device comprises a push element and the push element generates a gas to induce convective flow of the agent from the reservoir to the opposing surface via the population of passageway(s).
Embodiment 49. The device of any preceding Embodiment wherein the device further comprises a barrier element that has the capacity to independently separate the backing element from the reservoir, the backing element from the push element, the push element from the plurality of compartments, and/or the push element from the reservoir.
Embodiment 50. The device of any preceding Embodiment wherein the backing layer is covered by the enteric material.
Embodiment 51. The device of any preceding Embodiment wherein the backing layer is covered by the enteric material and the enteric material is substantially soluble at a pH greater than 5.5.
Embodiment 52. The device of any preceding Embodiment wherein the backing layer is covered by the enteric material and the enteric material is substantially soluble at a pH greater than 6.5.
Embodiment 53. The device of any preceding Embodiment wherein the backing layer is covered by the enteric material and the enteric material is substantially soluble at a pH in the range of about 5.5 to about 6.0.
Embodiment 54. The device of any preceding Embodiment wherein the backing layer is covered by the enteric material and the enteric material is substantially soluble at a pH in the range of about 6.0 to about 6.5.
Embodiment 55. The device of any preceding Embodiment wherein the backing layer is covered by the enteric material and the enteric material is substantially soluble at a pH in the range of about 6.5 to about 7.0.
Embodiment 56. The device of any preceding Embodiment wherein the backing layer is covered by the enteric material and the enteric material is substantially soluble at a pH in the range of about 7.0 to about 7.5.
Embodiment 57. The device of any preceding Embodiment wherein the enteric material further comprises a passageway-forming component.

## Claims

1. A device for the delivery of an agent to an intestinal site, the device comprising a backing element, a mucoadhesive layer for adhering the device to the intestinal site, and a reservoir comprising the agent, wherein
the mucoadhesive layer comprises a polymer, an opposing surface having the capacity to adhere to the intestinal site, and a population of passageway(s) formed through the mucoadhesive layer and extending from the reservoir to the opposing surface for delivery of the agent from the reservoir to the intestinal site, the mucoadhesive layer having a thickness of about 50 nm to about 10 mm,
wherein:
i) each of the passageway(s) has a minimum diameter greater than 10 microns, the diameter being determined by cryogenic scanning electron microscopy after 30 minutes of hydration at 20 °C in phosphate buffered saline at pH 6.5; or
ii) the device comprises a push element for induction of convective flow of the agent to the intestinal site, wherein
the agent comprises molecules having a molecular weight of at least 100 Da, the push element comprises an osmagent, and said passageways are permeable to said molecules; or
iii) the agent comprises a population of particles having a weight average particle size, P_{avg}, and
wherein the population has a number average minimum diameter, D_{avg}, of at least 10 microns, and the ratio of D_{avg} to P_{avg} is at least 2; or
iv) the agent comprises a population of particles having a weight average particle size, P_{avg}, of at least 50 nm, and
wherein the population has at least one member having a minimum diameter of at least 10 microns.

2. The device of the preceding claim wherein the release rate of the agent is:
(i) at least 20% within 2 hours in a USP Dissolution Assay 711 with Apparatus 1 and a dissolution medium of 150 mM phosphate buffered saline at pH 6.5; and/or
(ii) at least 50% within 2 hours in a USP Dissolution Assay 711 with Apparatus 1 and a dissolution medium of 150 mM phosphate buffered saline at pH 6.5; and/or
(iii) at least 80% within 2 hours in a USP Dissolution Assay 711 with Apparatus 1 and a dissolution medium of 150 mM phosphate buffered saline at pH 6.5; and/or
(iv) at least 90% within 2 hours in a USP Dissolution Assay 711 with Apparatus 1 and a dissolution medium of 150 mM phosphate buffered saline at pH 6.5; and/or
(v) at least 95% within 2 hours in a USP Dissolution Assay 711 with Apparatus 1 and a dissolution medium of 150 mM phosphate buffered saline at pH 6.5; and/or
(vi) at least 99% within 2 hours in a USP Dissolution Assay 711 with Apparatus 1 and a dissolution medium of 150 mM phosphate buffered saline at pH 6.5; and/or
(vii) at least 99.9% within 2 hours in a USP Dissolution Assay 711 with Apparatus 1 and a dissolution medium of 150 mM phosphate buffered saline at pH 6.5.

3. The device as in any preceding claim wherein the opposing surface adheres to the intestinal site with a tensile adhesive strength of at least about 10 mN when measured using an adapted porcine tissue tensile assay, and/or wherein the mucoadhesive layer has an average dry thickness of less than about 100 µm to less than about 800 µm, measured in a direction perpendicular to the opposing surface.

4. The device of any preceding claim wherein:
(i) a fraction of the passageway population members provide a substantially linear pathway from the reservoir to the opposing surface; and/or
(ii) a fraction of the passageway population members provide a tortuous pathway from the reservoir to the opposing surface; and/or
(iii) the passageway population is a population having only one member; and/or
(iv) the passageway population of the device according to any one of claims 1 to 5 comprises between 1 and 100 members;
(v) the passageway population of the device according to any one of claims 1 to 5 comprises at least 100 members; and/or
(vi) the device comprises an enteric material further comprising a passageway-forming component.

5. The device of any preceding claim wherein the population members are covered by an enteric material that inhibits release of the agent from the device before the device reaches the intestinal site and the enteric material is:
(i) substantially insoluble at a pH less than 5.5; or
(ii) substantially insoluble at a pH less than 6.0; or
(iii) substantially insoluble at a pH less than 6.5; or
(iv) substantially insoluble at a pH less than 7.0; or
(v) substantially insoluble at a pH less than 7.5.

6. The device of any preceding claim wherein the device has an outer surface having a total surface area of about 1 to about 10 cm² and/or wherein the opposing surface has a surface area of about 0.5 to about 1 cm², and/or wherein the device has a thickness in the range of 100 microns to 5 mm.

7. The device of any preceding claim wherein the device has an outer surface having a total surface area and the opposing surface comprises:
(i) about 30% of the total surface area of the device; or
(ii) about 40% of the total surface area of the device; or
(iii) about 50% of the total surface area of the device; or
(iv) about 60% of the total surface area of the device; or
(v) about 70% of the total surface area of the device; or
(vi) about 80% of the total surface area of the device; or
(vii) about 90% of the total surface area of the device; or
(viii) about 95% of the total surface area of the device; or
(ix) about 99% of the total surface area of the device.

8. The device of any preceding claim wherein the reservoir further comprises an excipient and the excipient is selected from the group consisting of stabilizers, glidants, bulking agents, anti-adherents, disintegrants, binders, sorbents, preservatives, cryoprotectants, permeation enhancers, hydrating agents, enzyme inhibitors, and mucus modifying agents, and/or wherein the device comprises an osmagent and the osmagent is selected from the group consisting of water-soluble salts, carbohydrates, small molecules, amino acids, and hydrogel forming polymers.

9. The device of any preceding claim wherein the mucoadhesive layer has a swelling ratio of:
(i) at most 1.05 as measured by cryo-SEM and SEM; or
(ii) at most 1.1 as measured by cryo-SEM and SEM; or
(iii) at most 1.2 as measured by cryo-SEM and SEM; or
(iv) at most 1.5 as measured by cryo-SEM and SEM; or
(v) at most 2 as measured by cryo-SEM and SEM.

10. The device of any preceding claim wherein the device comprises a push element, the push element has a swelling ratio of at least 2 to at least 4 as measured by cryo-SEM and SEM, and/or wherein the push element and the mucoadhesive layer are on opposing sides of the reservoir and an osmotic pressure is generated to induce convective flow of the agent from the reservoir to the opposing surface via the population of passageway(s), and/or wherein the push element comprises a plurality of compartments configured for controlled induction of convective flow of the agent through the population of passageway(s) to the opposing surface, and/or wherein the device comprises a push element and the push element generates a gas to induce convective flow of the agent from the reservoir to the opposing surface via the population of passageway(s).

11. The device of any preceding claim wherein the device further comprises a barrier element that has the capacity to independently separate the backing element from the reservoir, the backing element from the push element, the push element from the plurality of compartments, and/or the push element from the reservoir.

12. The device of any preceding claim wherein the backing layer is covered by the enteric material and the enteric material is:
(i) substantially soluble at a pH greater than 5.5; or
(ii) substantially soluble at a pH greater than 6.5; or
(iii) substantially soluble at a pH in the range of about 5.5 to about 6.0; or
(iv) substantially soluble at a pH in the range of about 6.0 to about 6.5; or
(v) substantially soluble at a pH in the range of about 6.5 to about 7.0; or
(vi) substantially soluble at a pH in the range of about 7.0 to about 7.5.

## Patentansprüche

1. Vorrichtung für die Abgabe eines Mittels an einer intestinalen Stelle, wobei die Vorrichtung Folgendes umfasst: ein stützendes Element, eine mucoadhäsive Schicht für die Haftung der Vorrichtung an der intestinalen Stelle und einen Behälter, der das Mittel umfasst, wobei
die mucoadhäsive Schicht Folgendes umfasst: ein Polymer, eine gegenüberliegende Oberfläche, die die Fähigkeit zur Haftung an der intestinalen Stelle aufweist, und eine Population von Durchgängen, die durch die mucoadhäsive Schicht ausgebildet sind und sich von dem Behälter zu der gegenüberliegenden Oberfläche für die Abgabe des Mittels aus dem Behälter an der intestinalen Stelle erstrecken, wobei die mucoadhäsive Schicht eine Dicke von etwa 50 nm bis etwa 10 mm aufweist,
wobei:
i) jeder der Durchgänge einen minimalen Durchmesser von größer als 10 Mikron aufweist, wobei der Durchmesser durch kryogene Rasterelektronenmikroskopie nach 30 Minuten Hydratation bei 20 °C in phosphatgepufferter Kochsalzlösung bei pH 6,5 bestimmt wird; oder
ii) die Vorrichtung ein Schiebeelement für die Induzierung eines Konvektionsstroms des Mittels zu der intestinalen Stelle umfasst, wobei das Mittel Moleküle umfasst, die ein Molekulargewicht von mindestens 100 Da aufweisen, das Schiebeelement ein osmotisch wirkendes Mittel umfasst und die Durchgänge für die Moleküle durchlässig sind; oder
iii) das Mittel eine Population von Partikeln umfasst, die eine gewichtsmittlere Partikelgröße, P_{avg}, aufweisen und
wobei die Population einen minimalen zahlenmittleren Durchmesser, D_{avg}, von mindestens 10 Mikron aufweist und das Verhältnis von D_{avg} zu P_{avg} mindestens 2 beträgt; oder
iv) das Mittel eine Population von Partikeln umfasst, die eine gewichtsmittlere Partikelgröße, P_{avg}, von mindestens 50 nm aufweist, und
wobei die Population mindestens ein Element aufweist, das einen minimalen Durchmesser von mindestens 10 Mikron aufweist.

2. Vorrichtung nach dem vorstehenden Anspruch, wobei die Freisetzungsrate des Mittels Folgende ist:
(i) mindestens 20 % innerhalb von 2 Stunden in einem USP Dissolution Assay (Auflösungstest) 711 mit Apparatur 1 und einem Auflösungsmedium von 150 mM phosphatgepufferter Kochsalzlösung bei pH 6,5; und/oder
(ii) mindestens 50 % innerhalb von 2 Stunden in einem USP Dissolution Assay 711 mit Apparatur 1 und einem Auflösungsmedium von 150 mM phosphatgepufferter Kochsalzlösung bei pH 6,5; und/oder
(iii) mindestens 80 % innerhalb von 2 Stunden in einem USP Dissolution Assay 711 mit Apparatur 1 und einem Auflösungsmedium von 150 mM phosphatgepufferter Kochsalzlösung bei pH 6,5; und/oder
(iv) mindestens 90 % innerhalb von 2 Stunden in einem USP Dissolution Assay 711 mit Apparatur 1 und einem Auflösungsmedium von 150 mM phosphatgepufferter Kochsalzlösung bei pH 6,5; und/oder
(v) mindestens 95 % innerhalb von 2 Stunden in einem USP Dissolution Assay 711 mit Apparatur 1 und einem Auflösungsmedium von 150 mM phosphatgepufferter Kochsalzlösung bei pH 6,5; und/oder
(vi) mindestens 99 % innerhalb von 2 Stunden in einem USP Dissolution Assay 711 mit Apparatur 1 und einem Auflösungsmedium von 150 mM phosphatgepufferter Kochsalzlösung bei pH 6,5; und/oder
(vii) mindestens 99,9 % innerhalb von 2 Stunden in einem USP Dissolution Assay 711 mit Apparatur 1 und einem Auflösungsmedium von 150 mM phosphatgepufferter Kochsalzlösung bei pH 6,5.

3. Vorrichtung nach einem vorstehenden Anspruch, wobei die gegenüberliegende Oberfläche an der intestinalen Stelle mit einer Haftzugfestigkeit von mindestens etwa 10 mN haftet, wenn unter Verwendung eines angepassten Zugfestigkeitstests für Schweinegewebe gemessen wird, und/oder wobei die mucoadhäsive Schicht eine durchschnittliche Trockendicke von weniger als etwa 100 µm bis weniger als etwa 800 µm, gemessen in einer Richtung senkrecht zu der gegenüberliegenden Oberfläche, aufweist.

4. Vorrichtung nach einem vorstehenden Anspruch, wobei:
(i) ein Bruchteil der Durchgangspopulationselemente einen im Wesentlichen linearen Weg von dem Behälter zu der gegenüberliegenden Oberfläche bereitstellen; und/oder
(ii) ein Bruchteil der Durchgangspopulationselemente einen gewundenen Weg von dem Behälter zu der gegenüberliegenden Oberfläche bereitstellen; und/oder
(iii) die Durchgangspopulation eine Population ist, die nur ein Element aufweist; und/oder
(iv) die Durchgangspopulation der Vorrichtung nach einem der Ansprüche 1 bis 5 zwischen 1 und 100 Elemente umfasst;
(v) die Durchgangspopulation der Vorrichtung nach einem der Ansprüche 1 bis 5 mindestens 100 Elemente umfasst; und/oder
(vi) die Vorrichtung ein enterisches Material umfasst, das weiter eine Durchgangbildende Komponente umfasst.

5. Vorrichtung nach einem vorstehenden Anspruch, wobei die Populationselemente von einem enterischen Material bedeckt sind, das die Freisetzung des Mittels aus der Vorrichtung verhindert, bevor die Vorrichtung die intestinale Stelle erreicht, und das enterische Material Folgendes ist:
(i) im Wesentlichen unlöslich bei einem pH von weniger als 5,5; oder
(ii) im Wesentlichen unlöslich bei einem pH von weniger als 6,0; oder
(iii) im Wesentlichen unlöslich bei einem pH von weniger als 6,5; oder
(iv) im Wesentlichen unlöslich bei einem pH von weniger als 7,0; oder
(v) im Wesentlichen unlöslich bei einem pH von weniger als 7,5.

6. Vorrichtung nach einem vorstehenden Anspruch, wobei die Vorrichtung eine äußere Oberfläche aufweist, die einen Gesamtoberflächenbereich von etwa 1 bis etwa 10 cm² aufweist, und/oder wobei die gegenüberliegende Oberfläche einen Oberflächenbereich von etwa 0,5 bis etwa 1 cm² aufweist und/oder wobei die Vorrichtung eine Dicke in dem Bereich von 100 Mikron bis 5 mm aufweist.

7. Vorrichtung nach einem vorstehenden Anspruch, wobei die Vorrichtung eine äußere Oberfläche aufweist, die einen Gesamtoberflächenbereich aufweist, und die gegenüberliegende Oberfläche Folgendes umfasst:
(i) etwa 30 % des Gesamtoberflächenbereichs der Vorrichtung; oder
(ii) etwa 40 % des Gesamtoberflächenbereichs der Vorrichtung; oder
(iii) etwa 50 % des Gesamtoberflächenbereichs der Vorrichtung; oder
(iv) etwa 60 % des Gesamtoberflächenbereichs der Vorrichtung; oder
(v) etwa 70 % des Gesamtoberflächenbereichs der Vorrichtung; oder
(vi) etwa 80 % des Gesamtoberflächenbereichs der Vorrichtung; oder
(vii) etwa 90 % des Gesamtoberflächenbereichs der Vorrichtung; oder
(viii) etwa 95 % des Gesamtoberflächenbereichs der Vorrichtung; oder
(ix) etwa 99 % des Gesamtoberflächenbereichs der Vorrichtung.

8. Vorrichtung nach einem vorstehenden Anspruch, wobei der Behälter weiter einen Hilfsstoff umfasst und der Hilfsstoff aus der Gruppe ausgewählt ist, die aus Folgenden besteht: Stabilisatoren, Gleitmitteln, Füllstoffen, Antihaftmitteln, Sprengmitteln, Bindemitteln, Sorptionsmitteln, Konservierungsmitteln, Kryoprotektiva, Durchlässigkeitsverstärkem, Hydratisierungmitteln, Enzyminhibitoren und Schleimmodifizierenden Mitteln, und/oder wobei die Vorrichtung ein osmotisch wirkendes Mittel umfasst und das osmotisch wirkende Mittel aus der Gruppe ausgewählt ist, die aus wasserlöslichen Salzen, Kohlenhydraten, kleinen Molekülen, Aminosäuren und Hydrogel-bildenden Polymeren besteht.

9. Vorrichtung nach einem vorstehenden Anspruch, wobei die mucoadhäsive Schicht ein Quellverhältnis von Folgendem aufweist:
(i) höchstens 1,05, wie durch Kryo-REM und REM gemessen wird; oder
(ii) höchstens 1,1, wie durch Kryo-REM und REM gemessen wird; oder
(iii) höchstens 1,2, wie durch Kryo-REM und REM gemessen wird; oder
(iv) höchstens 1,5, wie durch Kryo-REM und REM gemessen wird; oder
(v) höchstens 2, wie durch Kryo-REM und REM gemessen wird.

10. Vorrichtung nach einem vorstehenden Anspruch, wobei die Vorrichtung ein Schiebeelement umfasst, das Schiebeelement ein Quellverhältnis von mindestens 2 bis mindestens 4, wie durch Kryo-REM und REM gemessen wird, aufweist und/oder wobei das Schiebeelement und die mucoadhäsive Schicht auf gegenüberliegenden Seiten des Behälters vorliegen und ein osmotischer Druck erzeugt wird, um einen Konvektionsstrom des Mittels aus dem Behälter zu der gegenüberliegenden Oberfläche über die Population von Durchgängen zu induzieren, und/oder wobei das Schiebeelement eine Vielzahl von Kompartimenten umfasst, die zur geregelten Induzierung eines Konvektionsstroms des Mittels durch die Population von Durchgängen zu der gegenüberliegenden Oberfläche konfiguriert sind, und/oder wobei die Vorrichtung ein Schiebeelement umfasst und das Schiebeelement ein Gas erzeugt, um einen Konvektionsstrom des Mittels aus dem Behälter zu der gegenüberliegenden Oberfläche über die Population von Durchgängen zu induzieren.

11. Vorrichtung nach einem vorstehenden Anspruch, wobei die Vorrichtung weiter ein Barriereelement umfasst, das die Fähigkeit zur unabhängigen Trennung des stützenden Elements von dem Behälter, des stützenden Elements von dem Schiebeelement, des Schiebeelements von der Vielzahl von Kompartimenten und/oder des Schiebeelements von dem Behälter aufweist.

12. Vorrichtung nach einem vorstehenden Anspruch, wobei die stützende Schicht von dem enterischen Material bedeckt ist und das enterische Material Folgendes ist:
(i) im Wesentlichen löslich bei einem pH von größer als 5,5; oder
(ii) im Wesentlichen löslich bei einem pH von größer als 6,5; oder
(iii) im Wesentlichen löslich bei einem pH in dem Bereich von etwa 5,5 bis etwa 6,0; oder
(iv) im Wesentlichen löslich bei einem pH in dem Bereich von etwa 6,0 bis etwa 6,5; oder
(v) im Wesentlichen löslich bei einem pH in dem Bereich von etwa 6,5 bis etwa 7,0; oder
(vi) im Wesentlichen löslich bei einem pH in dem Bereich von etwa 7,0 bis etwa 7,5.

## Revendications

1. Dispositif destiné à l'administration d'un agent à un site intestinal, le dispositif comprenant un élément de support, une couche muco-adhésive pour faire adhérer le dispositif au site intestinal, et un réservoir comprenant l'agent, où
la couche muco-adhésive comprend un polymère, une surface opposée capable d'adhérer au site intestinal, et une population de passage(s) formée à travers la couche muco-adhésive et s'étendant à partir du réservoir vers la surface opposée pour l'administration de l'agent du réservoir vers le site intestinal, la couche muco-adhésive ayant une épaisseur allant d'environ 50 nm à environ 10 mm,
où :
i) chacun parmi le ou les passages possède un diamètre minimum supérieur à 10 microns, le diamètre étant déterminé par microscopie électronique à balayage cryogénique après 30 minutes d'hydratation à 20°C dans une solution saline tamponnée au phosphate à pH 6,5 ; ou
ii) le dispositif comprend un élément de poussée pour l'induction d'un flux convectif de l'agent vers le site intestinal, où
l'agent comprend des molécules ayant un poids moléculaire d'au moins 100 Da, l'élément de poussée comprend un agent osmotique, et lesdits passages sont perméables auxdites molécules ; ou
iii) l'agent comprend une population de particules ayant une taille de particules moyenne en poids, P_{moy}, et
où la population possède un diamètre minimum moyen en nombre, D_{moy}, d'au moins 10 microns, et le rapport de D_{moy} à P_{moy} est d'au moins 2 ; ou
iv) l'agent comprend une population de particules ayant une taille de particules moyenne en poids, P_{moy}, d'au moins 50 nm, et
où la population possède au moins un élément ayant un diamètre minimum d'au moins 10 microns.

2. Dispositif selon la revendication précédente, dans lequel la vitesse de libération de l'agent est :
(i) d'au moins 20% dans les 2 heures dans un Dosage de Dissolution USP 711 avec un Appareil 1 et un milieu de dissolution à base d'une solution saline tamponnée au phosphate à 150 mM et pH 6,5 ; et/ou
(ii) d'au moins 50% dans les 2 heures dans un Dosage de Dissolution USP 711 avec un Appareil 1 et un milieu de dissolution à base d'une solution saline tamponnée au phosphate à 150 mM et pH 6,5 ; et/ou
(iii) d'au moins 80% dans les 2 heures dans un Dosage de Dissolution USP 711 avec un Appareil 1 et un milieu de dissolution à base d'une solution saline tamponnée au phosphate à 150 mM et pH 6,5 ; et/ou
(iv) d'au moins 90% dans les 2 heures dans un Dosage de Dissolution USP 711 avec un Appareil 1 et un milieu de dissolution à base d'une solution saline tamponnée au phosphate à 150 mM et pH 6,5 ; et/ou
(v) d'au moins 95% dans les 2 heures dans un Dosage de Dissolution USP 711 avec un Appareil 1 et un milieu de dissolution à base d'une solution saline tamponnée au phosphate à 150 mM et pH 6,5 ; et/ou
(vi) d'au moins 99% dans les 2 heures dans un Dosage de Dissolution USP 711 avec un Appareil 1 et un milieu de dissolution à base d'une solution saline tamponnée au phosphate à 150 mM et pH 6,5 ; et/ou
(vii) d'au moins 99,9% dans les 2 heures dans un Dosage de Dissolution USP 711 avec un Appareil 1 et un milieu de dissolution à base d'une solution saline tamponnée au phosphate à 150 mM et pH 6,5.

3. Dispositif selon l'une quelconque des revendications précédentes, dans lequel la surface opposée adhère au site intestinal avec une résistance à l'arrachement d'au moins environ 10 mN, lors d'une mesure à l'aide d'un essai de traction sur tissu porcin adapté, et/ou où la couche muco-adhésive possède une épaisseur sèche moyenne allant de moins d'environ 100 µm à moins d'environ 800 µm, mesurée dans un sens perpendiculaire par rapport à la surface opposée.

4. Dispositif selon l'une quelconque des revendications précédentes, dans lequel
(i) une fraction des éléments de la population de passages fournit un chemin substantiellement linéaire du réservoir vers la surface opposée ; et/ou
(ii) une fraction des éléments de la population de passages fournit un chemin tortueux du réservoir vers la surface opposée ; et/ou
(iii) la population de passages est une population n'ayant qu'un seul élément ; et/ou
(iv) la population de passages du dispositif selon l'une quelconque des revendications 1 à 5 comprend entre 1 et 100 éléments ;
(v) la population de passages du dispositif selon l'une quelconque des revendications 1 à 5 comprend au moins 100 éléments ; et/ou
(vi) le dispositif comprend un matériau entérique comprenant en outre un composant formateur de passages.

5. Dispositif selon l'une quelconque des revendications précédentes, dans lequel les éléments de population sont revêtus par un matériau entérique qui inhibe la libération de l'agent à partir du dispositif avant que le dispositif n'atteigne le site intestinal et le matériau entérique est :
(i) sensiblement insoluble à un pH inférieur à 5,5 ; ou
(ii) sensiblement insoluble à un pH inférieur à 6,0 ; ou
(iii) sensiblement insoluble à un pH inférieur à 6,5 ; ou
(iv) sensiblement insoluble à un pH inférieur à 7,0 ; ou
(v) sensiblement insoluble à un pH inférieur à 7,5.

6. Dispositif selon l'une quelconque des revendications précédentes, le dispositif présentant une surface externe ayant une aire totale allant d'environ 1 à environ 10 cm² et/ou où la surface opposée possède une aire allant d'environ 0,5 à environ 1 cm² et/ou où le dispositif possède une épaisseur dans la plage allant de 100 microns à 5 mm.

7. Dispositif selon l'une quelconque des revendications précédentes, le dispositif présentant une surface externe ayant une aire totale et la surface opposée comprend :
(i) environ 30% de l'aire totale du dispositif ; ou
(ii) environ 40% de l'aire totale du dispositif ; ou
(iii) environ 50% de l'aire totale du dispositif ; ou
(iv) environ 60% de l'aire totale du dispositif ; ou
(v) environ 70% de l'aire totale du dispositif ; ou
(vi) environ 80% de l'aire totale du dispositif ; ou
(vii) environ 90% de l'aire totale du dispositif ; ou
(viii) environ 95% de l'aire totale du dispositif ; ou
(ix) environ 99% de l'aire totale du dispositif.

8. Dispositif selon l'une quelconque des revendications précédentes, dans lequel le réservoir comprend en outre un excipient et l'excipient est choisi dans le groupe constitué par les stabilisants, les agents glissants, les agents diluants, les antiadhérents, les délitants, les liants, les sorbants, les conservateurs, les cryoprotecteurs, les améliorateurs de perméation, les agents hydratants, les inhibiteurs enzymatiques, et les agents modificateurs de mucus, et/ou où le dispositif comprend un agent osmotique et l'agent osmotique est choisi dans le groupe constitué par les sels hydrosolubles, les glucides, les petites molécules, les acides aminés, et les polymères formateurs d'hydrogel.

9. Dispositif selon l'une quelconque des revendications précédentes, dans lequel la couche muco-adhésive possède un taux de gonflement
(i) d'au plus 1,05, tel que mesuré par cryo-MEB et MEB ; ou
(ii) d'au plus 1,1, tel que mesuré par cryo-MEB et MEB ; ou
(iii) d'au plus 1,2, tel que mesuré par cryo-MEB et MEB ; ou
(iv) d'au plus 1,5, tel que mesuré par cryo-MEB et MEB ; ou
(v) d'au plus 2, tel que mesuré par cryo-MEB et MEB.

10. Dispositif selon l'une quelconque des revendications précédentes, le dispositif comprenant un élément de poussée, l'élément de poussée présentant un taux de gonflement allant d'au moins 2 à au moins 4, tel que mesuré par cryo-MEB et MEB, et/ou où l'élément de poussée et la couche muco-adhésive se trouvent sur des côtés opposés du réservoir et une pression osmotique est générée afin d'induire un flux convectif de l'agent du réservoir vers la surface opposée via la population de passage(s), et/ou où l'élément de poussée comprend une pluralité de compartiments configurés pour une induction contrôlée d'un flux convectif de l'agent à travers la population de passage(s) vers la surface opposée, et/ou où le dispositif comprend un élément de poussée, et l'élément de poussée génère un gaz afin d'induire un flux convectif de l'agent du réservoir vers la surface opposée via la population de passage(s).

11. Dispositif selon l'une quelconque des revendications précédentes, le dispositif comprenant en outre un élément de barrière capable de séparer indépendamment l'élément de support du réservoir, l'élément de support de l'élément de poussée, l'élément de poussée de la pluralité de compartiments, et/ou l'élément de poussée du réservoir.

12. Dispositif selon l'une quelconque des revendications précédentes, dans lequel la couche de support est revêtue par le matériau entérique et le matériau entérique est
(i) sensiblement soluble à un pH supérieur à 5,5 ; ou
(ii) sensiblement soluble à un pH supérieur à 6,5 ; ou
(iii) sensiblement soluble à un pH dans la plage allant d'environ 5,5 à environ 6,0 ; ou
(iv) sensiblement soluble à un pH dans la plage allant d'environ 6,0 à environ 6,5 ; ou
(v) sensiblement soluble à un pH dans la plage allant d'environ 6,5 à environ 7,0 ; ou
(vi) sensiblement soluble à un pH dans la plage allant d'environ 7,0 à environ 7,5.
